(19) Europäisches Patentamt
European Patent Office
Office européen des brevets

(11) **EP 2 833 126 A1**

(12) **EUROPEAN PATENT APPLICATION**
published in accordance with Art. 153(4) EPC

(43) Date of publication:
**04.02.2015 Bulletin 2015/06**

(21) Application number: **13767259.8**

(22) Date of filing: **29.03.2013**

(51) Int Cl.:
**G01N 27/00** (2006.01)   **C12N 15/00** (2006.01)

(86) International application number:
**PCT/JP2013/059645**

(87) International publication number:
**WO 2013/147208 (03.10.2013 Gazette 2013/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **29.03.2012 JP 2012077975**

(71) Applicant: **Osaka University**
**Suita-shi, Osaka 565-0871 (JP)**

(72) Inventors:
• **KAWAI, Tomoji**
**Suita-shi**
**Osaka 565-0871 (JP)**
• **OHSHIRO, Takahito**
**Suita-shi**
**Osaka 565-0871 (JP)**
• **MATSUBARA, Kazuki**
**Suita-shi**
**Osaka 565-0871 (JP)**
• **FURUHASHI, Masayuki**
**Suita-shi**
**Osaka 565-0871 (JP)**
• **TSUTSUI, Makusu**
**Suita-shi**
**Osaka 565-0871 (JP)**
• **TANIGUCHI, Masateru**
**Suita-shi**
**Osaka 565-0871 (JP)**

(74) Representative: **Jones, Helen M.M.**
**Gill Jennings & Every LLP**
**The Broadgate Tower**
**20 Primrose Street**
**London EC2A 2ES (GB)**

(54) **METHOD FOR DETERMINING POLYNUCLEOTIDE BASE SEQUENCE AND DEVICE FOR DETERMINING POLYNUCLEOTIDE BASE SEQUENCE**

(57)   A maximum current value and pulse continuation duration are measured for each of plural pulses of tunnel current arising as a polynucleotide passes through between an electrode pair, and the polynucleotide base sequence is determined based on the maximum current value and the pulse continuation duration.

FIG.5

EP 2 833 126 A1

**Description**

Technical Field

The present invention relates to a polynucleotide base sequence determination method and to a polynucleotide base sequence determination device. More specifically, it relates to a polynucleotide base sequence determination method and to a polynucleotide base sequence determination device based on tunnel current arising as a polynucleotide passes between an electrode pair.

Background Art

Technology to analyze polynucleotide base sequences is not simply limited to the academic research field, but is applied in fields ranging from medicine, to drug discovery and criminal forensics, and there is increasing interest in developments in this technology.

Conventional polynucleotide (specifically DNA) sequencers employ optical measuring technology to identify fluorescent markers, rather than directly identifying the nucleotides themselves that configure the polynucleotides. This is because, when trying to analyze base sequences of a polynucleotide with a conventional sequencer, even when PCR is performed with the polynucleotide as a template, fluorescent markers need to be added to polynucleotides that have been elongated by PCR. Such operations not only require the use of many reagents, but also need a lot of time. This means that polynucleotide base sequence analysis using a conventional sequencer requires significant funding and time.

Over the last couple decades there have been attempts to develop technology to directly analyze the nucleotides configuring a polynucleotide by employing one molecule of a polynucleotide.

For example, there have been attempts to develop technology to analyze a polynucleotide base sequence by detecting an ion current using nanoscale pores (referred to below as "nanopores") of chemically designed $\alpha$-hemolysin (see Non-Patent Documents 1 to 5). However, such technology has many issues such as (1) that there are limitations to pore size selection, and (2) that the system is unstable, and there are no prospects of the system being put into practice.

It is against this backdrop that new attempts are being made to analyze polynucleotide base sequences based on "tunnel current" arising when a polynucleotide passes between an extremely narrow electrode pair (see, for example, Patent Document 1 and Non-Patent Document 6). Such technology is technology that analyzes polynucleotide base sequences based on tunnel current, which is a completely different current from an "ion current", and is technology that takes a completely different approach from the technology described in Non-Patent Documents 1 to 5.

Patent Documents

Patent Document 1: WO2011/1085404A1 (Laid-Open Date: September 9, 2011) Non-Patent Documents
Non-patent document 1: J. Li, D. Stein, C. McMullan, D. Branton, M. J. Aziz, J. A. Golovchenko, Nature 412, 166 (2001)
Non-patent document 2: A. J. Storm, J. H. Chen, X. S. Ling, H. W. Zandbergen, C. Dekker, Nature Mat. 2, 537 (2003)
Non-patent document 3: C. Dekker, Nat. Nanotechnol. 2, 209 (2007)
Non-patent document 4: D. Branton, D. W. Deamer, A. Marziali, H. Bayley, S. A. Benner, T. Butler, M. Di Ventra, S. Garaj, A. Hibbs, X. Huang, S. B. Jovanovich, P. S. Krstic, S. Lindsay, X. S. Ling, C. H. Mastrangelo, A. Meller, J. S. Oliver, Y. V. Pershin, J. M. Ramsey, R. Riehn, G. V. Soni, V. Tabard-Cossa, M. Wanunu, M. Wiggin, J. A. Schloss, Nat. Biotech. 26, 1146 (2008)
Non-patent document 5: M. Zwolak, M. Di Ventra, Rev. Mod. Phys. 80, 141 (2008)
Non-patent document 6: Nature Nanotechnology, 2010, April, 5 (4), 286 to 290.

DISCLOSURE OF INVENTION

Technical Problem

However, although the technology for analyzing polynucleotide base sequences based on tunnel currents described above is appropriate for determining the base sequence of nucleotides or short polynucleotides, there has been no method established for determining base sequences of long polynucleotides using this technology, and this is why there is an urgent demand to establish such a method.

In consideration of the above circumstances, an object of the present invention is to establish a method and device to determine a long polynucleotide base sequence using technology to analyze base sequences of polynucleotides based on tunnel current.

Solution to Problem

In order to address the above issues, a polynucleotide base sequence determination method of the present invention includes: a first process of passing a polynucleotide between an electrode pair; a second process of detecting plural pulses of tunnel current arising as the polynucleotide passes between the electrode pair, and of measuring a maximum current value and pulse continuation duration for each of the plural pulses; a third process of generating primary base sequence data in which each of the plural pulses is associated with a specific type of nucleotide by comparing a magnitude order between the maximum current values of the plural pulses and a magnitude order between reference current values corresponding to electron states caused by energy level differences between individual nucleotides and metal configuring the electrode pair; a fourth process of extracting from among the plural pulses a pulse group configured by pulses with successive pulse continuation durations, and of extracting from among the primary base sequence data plural secondary base sequence data corresponding to the pulse group; a fifth process of searching the plural secondary base sequence data for base sequences that are common across at least two of the secondary base sequence data; and a sixth process of joining together the secondary base sequence data that have the common base sequence via the common base sequence.

In order to address the above issues, a polynucleotide base sequence determination device of the present invention includes: an electrode pair that has an inter-electrode distance through which a polynucleotide is capable of passing; a measurement section that detects plural pulses of tunnel current arising as the polynucleotide passes between the electrode pair, and that measures a maximum current value and pulse continuation duration for each of the plural pulses; a primary base sequence data generating section that generates primary base sequence data in which each of the plural pulses is associated with a specific type of nucleotide by comparing a magnitude order between the maximum current values of the plural pulses and a magnitude order between reference current values corresponding to electron states caused by energy level differences between individual nucleotides and metal configuring the electrode pair; a secondary base sequence data extraction section that extracts from among the plural pulses a pulse group configured by pulses with successive pulse continuation durations, and that extracts from among the primary base sequence data plural secondary base sequence data corresponding to the pulse group; a common sequence search section that searches the plural secondary base sequence data for base sequences that are common across at least two of the secondary base sequence data; and a sequence data connection section that joins together the secondary base sequence data that have the common base sequence via the common base sequence.

Advantageous Effects of Invention

The present invention exhibits the advantageous effect of not only obviously allowing base sequence data to be read directly from polynucleotides such as DNA, but also allowing base sequence data to be read directly from polynucleotides such as RNA.

The present invention not only allows reduction in the effort required in conventional technology in the extraction and purifying operations of polynucleotides (for example, DNA), but also allows PCR reactions using the polynucleotide to be omitted, and accordingly exhibits the advantageous effect of allowing polynucleotide base sequence determination to be performed easily in a short period of time.

The present invention does not need to employ processing to break up a polynucleotide, such as that employed in a conventional shotgun sequencing method, and therefore exhibits the advantageous effect of allowing polynucleotide base sequence determination to be performed easily in a short period of time.

The present invention exhibits the advantageous effect of allowing a base sequence to be determined even for a modified polynucleotide or a damaged polynucleotide.

The present invention exhibits the advantageous effect of allowing genetic expression data and epigenetic data due to ageing or illness to be directly obtained from a polynucleotide.

The present invention exhibits the advantageous effect of allowing base sequence determination to be made even when there is a mere trace of a polynucleotide (for example one molecule of DNA or RNA).

The present invention does not require a biomolecule, and determines base sequences by employing electrodes with high mechanical strength, and accordingly exhibits the advantageous effect of allowing stable polynucleotide base sequence determination to be performed.

The present invention exhibits the advantageous effect of allowing stable polynucleotide base sequence determination to be performed even, for example, under conditions that would change biomolecules (for example, high temperature conditions that would sever hydrogen bonds formed between molecules of DNA or RNA).

The present invention does not require a device for actively inserting a polynucleotide between the electrodes or a device for cleaning the electrodes, and thereby exhibits the advantageous effects of allowing a polynucleotide base sequence to be determined by a compact device, and also allowing low cost polynucleotide base sequence determination to be performed.

BRIEF DESCRIPTION OF DRAWINGS

Fig. 1 is a schematic diagram illustrating a polynucleotide passing between an electrode pair.

Fig. 2 shows graphs illustrating various data of an exemplary embodiment of the present invention.

Fig. 3 shows graphs illustrating various data of an exemplary embodiment of the present invention.

Fig. 4 shows graphs illustrating various data of an exemplary embodiment of the present invention.

Fig. 5 is a block diagram illustrating an example of a configuration of a polynucleotide base sequence determining device of an exemplary embodiment of the present invention.

Fig. 6 is a flow chart illustrating an example of operation of a polynucleotide base sequence determining device of an exemplary embodiment of the present invention.

DESCRIPTION OF EMBODIMENTS

Explanation follows regarding an exemplary embodiment of the present invention, however the present invention is not limited thereby.

1. Polynucleotide Base Sequence Determination Method

A polynucleotide base sequence determination method of the present invention includes the following first process to sixth process. Namely:

a first process: a process of passing a polynucleotide between an electrode pair;

a second process: a process of detecting plural pulses of tunnel current arising as the polynucleotide passes through between the electrode pair, and of measuring the maximum current value and pulse continuation duration for each of the plural pulses;

a third process: a process of generating primary base sequence data in which each of the plural pulses is associated with a specific type of nucleotide by comparing a magnitude order between the maximum current values of the plural pulses and a magnitude order between reference current values corresponding to electron states caused by energy level differences between individual nucleotides (called reference nucleotides) and metal configuring the electrode pair;

a fourth process: a process of extracting from out of the plural pulses a pulse group configured by pulses with successive pulse continuation durations, and of extracting from out of the primary base sequence data plural secondary base sequence data corresponding to the pulse group;

a fifth process: a process of searching the plural secondary base sequence data for base sequences that are common across at least two of the secondary base sequence data; and

a sixth process: a process of joining the secondary base sequence data that have the common base sequence together through the common base sequence.

Explanation follows regarding each of the processes.

1.1: First Process

The first process is a process in which a polynucleotide is passed between an electrode pair.

When employed within the present specification, the term "polynucleotide" is used interchangeably with the terms "oligonucleotide" and "gene", and is used to refer to a nucleotide polymer. Note that when used in the present specification, the term "oligonucleotides" is used to mean 2 to several tens of individual, and more specifically, 2 to 50 individual nucleotides. "Polynucleotides" is used to mean several tens of individual or more, and more specifically, more than 50 individual nucleotides.

There is no particular limitation to nucleotides configuring the polynucleotide referred to above, and they may be any given ribonucleotides, or they may be any given deoxyribonucleotide. Moreover, nucleotides configuring the polynucleotide referred to above may be chemically modified ribonucleotides or deoxyribonucleotides (for example subjected to methylation, oxylation, hydroxylation, formylation, carboxylation, dimerization, base-removal or the like).

Ribonucleotides are not particularly limited, and may include for example: adenosine monophosphate (rAMP), adenosine diphosphate (rADP), adenosine triphosphate (rATP), guanosine monophosphate (rGMP), guanosine diphosphate (rGDP), guanosine triphosphate (rGTP), cytidine monophosphate (rCMP), cytidine diphosphate (rCDP), cytidine triphosphate (rCTP), uridine monophosphate (rUMP), uridine diphosphate (rUDP), and uridine triphosphate (rUTP).

Deoxyribonucleotides are not particularly limited, and may include for example: deoxyadenosine monophosphate (dAMP), deoxyadenosine diphosphate (dADP), deoxyadenosine triphosphate (dATP), deoxyguanosine monophosphate (dGMP), deoxyguanosine diphosphate (dGDP), deoxyguanosine triphosphate (dGTP), deoxycytidine monophosphate (dCMP), deoxycytidine diphosphate (dCDP), deoxycytidine triphosphate (dCTP), deoxyuridine monophosphate (dUMP), deoxyuridine diphosphate (dUMP), deoxyuridine diphosphate (dUTP), deoxythymidine monophosphate (dTMP), deoxythymidine diphosphate (dTDP), and deoxythymidine triphosphate (dTTP).

There is no particular limitation to such chemically modified ribonucleotides or deoxyribonucleotides, and examples thereof include methylcytosine, methyladenine, oxoguanine, hydroxymethylcytosine, a thymine dimer, methyladenine, formylcytosine, and ribonucleotides or deoxyribonucleotides from which bases have been removed.

In the first process, the polynucleotide may be passed between the electrode pair by dissolving the above polynucleotide in a solvent, and filling the solution between the electrodes forming the electrode pair, or holding the electrode pair in the above solution.

There is no particular limitation to the solvent in which the nucleotide is dissolved, however for example ultrapure water may be employed. Ultrapure water can, for example, be produced employing a Milli-Q Integral 3 (device name) made by EMD Millipore Corporation (Milli-Q Integral 3/5/10/15 (catalogue number)). The concentration of the polynucleotide in the solution is not particularly limited and is for example 0.01 to $1.0\mu$M. Obviously a base sequence of a polynucleotide can be analyzed as long as there is one molecule of the polynucleotide present in the solution.

In the first process a voltage is applied across the electrode pair. By doing so, a tunnel current arises between the electrodes forming the electrode pair when the polynucleotide passes through between the electrode pair. The voltage that is applied is not particularly limited, and may be for example 0.25V to 0.75V.

In the first process, the above polynucleotide is passed between the electrode pair.

There is no particular limitation to a specific method for passing the polynucleotide between the electrode pair, and it is possible, for example, to make the polynucleotide move by thermal diffusion (or in other words Brownian motion) or by an alternating current, and to pass the polynucleotide between the electrode pair using this movement. Out of these, it is preferable to move the polynucleotide using thermal diffusion, and to pass the polynucleotide between the electrode pair using this movement. It is possible by adopting the above configuration to make the polynucleotide be present between the electrode pair over a long period of time, thereby enabling more data to be obtained on the partial sequence of the polynucleotide. As a result it is accordingly possible to determine longer polynucleotides and more precisely the base sequence of the polynucleotide.

There is no particular limitation to the temperature when performing thermal diffusion on the polynucleotide, and any suitable setting can be made therefor. For example, 5°C to 70°C is preferable, and 20°C to 50°C is more preferable.

In contrast to conventional technology, there is no need in the present invention for electrodes with pores formed from proteins, and hence there is no loss of electrode function when, for example, the polynucleotide is thermally diffused at high temperature. Moreover, as long as the polynucleotide is thermally diffused at high temperature, intra-molecular interaction of the polynucleotide (such as for example hydrogen bonding) can be prevented. Namely, by thermally diffusing the polynucleotide at high temperature, the polynucleotide can be prevented from forming double-strands. As a result, more accurate determination of base sequences of the polynucleotide is enabled.

Explanation next follows regarding the electrode pair employed in the present exemplary embodiment.

In order to implement the present exemplary embodiment a tunnel current needs to be generated between the electrode pair when the polynucleotide passes through. The distance between the electrode pair is important for generating such a tunnel current. Tunnel current does not readily flow between the electrode pair, or two or more polynucleotides enter between the electrode pair at the same time when the distance between the electrode pair is excessively longer than the molecular diameter of each of the nucleotides configuring the polynucleotide. However the polynucleotide no longer enters between the electrode pair in the opposite case when the distance between the electrode pair is excessively shorter than the molecular diameter of each of the nucleotides configuring the polynucleotide.

It becomes difficult to detect pulses caused by tunnel current across a single molecule of each of the nucleotides configuring the polynucleotide when the distance between the electrode pair is excessively longer or excessively shorter than the molecular diameter of each of the nucleotides configuring the polynucleotide. The distance between the electrodes configuring the electrode pair is hence preferably made slightly shorter than, or the same as, or slightly longer than the molecular diameter of each of the nucleotides configuring the polynucleotide. For example, the inter-electrode distance is a length that is 0.5 times to 2 times the molecular diameter of the nucleotides, with the inter-electrode distance preferably set at a length of 1 times to 1.5 times thereof, and more preferably set at a length of 1 times to 1.2 times.

Since the molecular diameter of the nucleotides is known to a person of skill in the art, appropriate selection of the optimum distance between the electrode pair can be made by a person of skill in the art by reading the present specification. For example, since the molecular diameter of nucleotides in a phosphate state is about 1nm, using this molecular diameter as a reference, the distance between the electrode pair is, for example, set at 0.5nm to 2nm, preferably at 1nm to 1.5nm and more preferably at 1m to 1.2nm.

Moreover, it is preferable that the above electrode pair maintains a constant inter-electrode distance (or is able to control the inter-electrode distance to a constant distance). Namely, the above electrode pair is preferably an electrode pair in which the inter-electrode distance does not change during measuring tunnel current.

For example, the proportional change in the inter-electrode distance is preferably 1% or less, more preferably 0.1 % or less, even more preferably 0.01 % or less, and most preferably 0.001% or less.

Electrode pairs fabricated using conventional technology seem at first glance to maintain a constant inter-electrode distance when observed with the naked eye, however there are actually microscopic changes in the inter-electrode distance. When there are even microscopic changes in the inter-electrode distance this leads to fluctuations in the tunnel current values. Namely, tunnel current values caused by the same substance vary, lowering the determination precision of polynucleotide base sequences.

However, as long as an electrode pair is employed that is capable of maintaining a constant inter-electrode distance, then even higher determination precision of polynucleotide base sequences can be achieved.

Note that such electrode pairs can be easily fabricated using technology invented by the present inventor (for example with a nanofabricated mechanically-controllable break junctions, as described later). Details regarding such technology are given later.

There is no particular limitation to a specific fabrication method for the above electrode pair. An example of a fabrication method is illustrated below.

The above electrode pairs can be easily fabricated using a known nanofabricated mechanically-controllable break junction method. A nanofabricated mechanically-controllable break junction method is an excellent method capable of controlling of the inter-electrode distance with excellent mechanical stability at resolution at the picometer level or finer. Fabrication methods for electrode pairs employing nanofabricated mechanically-controllable break junction methods are described for example in J. M. van Ruitenbeek, A. Alvarez, I. Pineyro, C. Grahmann, P. Joyez, M. H. Devoret, D. Esteve, C. Urbina, Rev. Sci. Instrum. 67, 108 (1996) and M. Tsutsui, K. Shoji, M. Taniguchi, T. Kawai, Nano Lett. 8, 345 (2008). An appropriate metal such as gold may be employed as the electrode material.

For example, the electrode pair may be fabricated using the process set out below.

Firstly, known electron beam lithography and lift-off technology is used to pattern form nanoscale gold junctions on a polyimide coated flexible metal substrate employing an electron beam lithography device (JEOL Ltd., catalogue number: JSM6500F). Next, polyamide beneath the junctions is removed by etching based on a known etching process (for example a reactive ion etching process) employing a reactive ion etching device (Samco Inc., catalogue number: 10NR).

A nanoscale gold bridge structure with a 3-point bent structure is then fabricated by bending the substrate. Here, precise bending of the substrate is performed employing a piezoelectric actuator (CEDRAT, catalogue number: APA150M), enabling the inter-electrode distance of the electrode pair to be controlled at resolution at the picometer level or finer.

Next, the fabricated bridge is pulled. A portion of the bridge is broken. The bridge is pulled further, and the size of the gap (inter-electrode distance) occurring due to the break is set to the length (about 1nm) of the target nucleotide molecule. Here, the inter-electrode distance of the electrode pair may be accurately controlled by regulating the bridge pulling employing self-breaking technology (see for example M. Tsutsui, K. Shoji, M. Taniguchi, T. Kawai, Nano Lett. 8, 345 (2008) and M. Tsutsui, M. Taniguchi, T. Kawai, Appl. Phys. Lett. 93, 163115 (2008)).

Specifically, a DC bias voltage ($V_b$) of 0.1V is applied to the bridge employing series resistance of 10kΩ at a programmed junction stretching speed, pulling the gold nanojunction, and breaking the bridge by a resistance feedback method (see M. Tsutsui, K. Shoji, M. Taniguchi, T. Kawai, Nano Lett. 8, 345 (2008), and M. Tsutsui, M. Taniguchi, T. Kawai, Appl. Phys. Lett. 93, 163115 (2008)) employing a data acquisition board (National Instruments Corporation, catalogue number: NI PCIe-6321). Next, the bridge is pulled further and the size of the gap (inter-electrode distance) occurring due to the break is set to the length of the target nucleotide molecule. The electrode pair is thereby formed.

1.2: Second Process

The second process is a process in which plural pulses of tunnel current arising as the polynucleotide passes between the electrode pair are detected, and the maximum current value and the pulse continuation duration are measured for each of the plural pulses.

There is no particular limitation to the number of pulses detected in the second process, and the greater the number thereof the better the determination precision of the full-length base sequence of the polynucleotide. Note that for example the period of time for measuring the tunnel current may be lengthened to increase the number of pulses detected. There is no particular limitation to the duration for measuring the tunnel current, and possible values thereof are 10 minutes, 20 minutes, 30 minutes, 40 minutes, 50 minutes and 1 hour. The above time may be appropriately set according to the length of the polynucleotides.

Explanation follows below regarding a specific tunnel current measurement method.

For example, a tunnel current caused by the nucleotides configuring the polynucleotide arises between the electrode pair when the polynucleotide passes between the electrode pair as long as the electrode pair is held in a solution in which the polynucleotide is dissolved and a voltage (for example 0.25V to 0.75V) is applied between the electrode pair. Explanation follows regarding the mechanism giving rise to the tunnel current (plural tunnel currents).

As the polynucleotide enters between the electrodes, firstly a given nucleotide configuring the polynucleotide (referred to below as the first nucleotide) is trapped between the electrodes. A tunnel current caused by the first nucleotide arises between the electrodes while the first nucleotide is trapped between the electrodes.

Note that the first nucleotide is sometimes a 5' terminal nucleotide of a polynucleotide, is sometimes a 3' terminal nucleotide of a polynucleotide, and is sometimes a nucleotide present between the 5' terminal and the 3' terminal.

Next, after the first nucleotide has completely passed through between the electrode pair, another nucleotide is trapped between the electrodes (referred to below as the second nucleotide). Tunnel current arises between the electrode pair caused by the second nucleotide while the second nucleotide is trapped between the electrode pair.

Note that the above second nucleotide is sometimes a nucleotide adjacent to the first nucleotide, and is sometimes a nucleotide that is not adjacent to the first nucleotide. It is possible to determine whether or not the second nucleotide is a nucleotide adjacent to the first nucleotide based on the pulse continuation duration, and explanation is given later regarding this point.

As described above, tunnel current caused by the nucleotides configuring the polynucleotide arises between the electrode pair.

Then, when the polynucleotide has passed through between the electrode pair (when the last nucleotide configuring the polynucleotide moves away from the electrode pair) the tunnel current arising between the electrodes disappears.

The measurement of the tunnel current arising between the electrode pair may be measured employing a known ammeter. The tunnel current signal may moreover be first amplified employing for example a current amplifier. Since employing a current amplifier enables amplification of weak tunnel current values, it is possible to measure the tunnel current with high sensitivity. An example of a current amplifier is a commercially available variable gain high speed current amplifier (Catalogue Number: DHPCA-100, manufactured by FEMTO Messtechnik GmbH).

The tunnel current pulses can accordingly be detected by measuring the tunnel current flowing between the electrode pair for a specific period of time, and serially determining whether or not the current value of the tunnel current exceeds a base level. Specifically, according to the determination referred to above, by identifying the time when the tunnel current exceeds the base level and by identifying the time when the tunnel current returns once again to the base level, the signal in the period between these two times can be detected as a tunnel current pulse caused by the nucleotide. Employing a graph expressing a relationship between the measured current value of the tunnel current and the tunnel current measurement duration (for example a curved line graph) enables such determination to be easily performed by visual inspection.

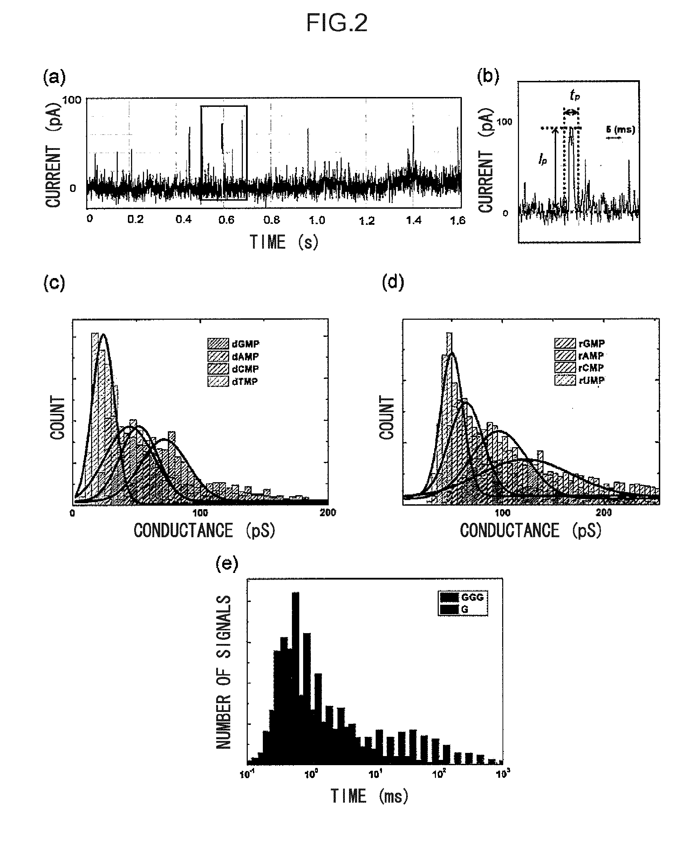

Fig. 2(b) illustrates an example of a tunnel current pulse. As illustrated in Fig. 2(b), the maximum current value (Ip) and the pulse continuation duration (tp) can be computed for each of the pulses from the graph that expresses the relationship between the measured current value of the tunnel current and the measured duration of the tunnel current.

1-3: Third Process

The third process is a process in which primary base sequence data is generated in which the each of the plural pulses is associated with a specific type of nucleotide by comparing a magnitude order between the maximum current values of the plural pulses, and a magnitude order between reference current values corresponding to electron states caused by energy level differences between individual nucleotides (referred to as reference nucleotides) and the metal configuring the electrode pair.

Moreover, the third process may be configured as a process in which primary base sequence data is generated in which the each of the plural pulses is associated with a specific type of nucleotide by comparing a magnitude order between maximum current values of plural pulses and a magnitude order between the maximum current values of individual nucleotides (referred to as reference nucleotides).

For example, say A, B, C and D are predetermined reference current values for each of 4 types of reference nucleotide of known structure, and their magnitude relationship is A < B < C < D. Then, say each of the measured maximum current values of the plural pulses can be classified as a, b, c, and d, and the magnitude relationship between these maximum current values is a < b < c < d.

In such a case, the nucleotide corresponding to the maximum current value a and the reference nucleotide corresponding to the reference current value A can be determined as being the same nucleotide, and the nucleotide corresponding to the maximum current value b and the reference nucleotide corresponding to the reference current value B can be determined as being the same nucleotide, and the nucleotide corresponding to the maximum current value c and the reference nucleotide corresponding to the reference current value C can be determined as being the same nucleotide, and the nucleotide corresponding to the maximum current value d and the reference nucleotide corresponding to the reference current value D can be determined as being the same nucleotide.

Since the structure of the reference nucleotide is known it becomes possible to associate each of the measured pulses with a specific type of nucleotide.

Note that the third process described above may include a process of determining whether or not there are the same number of types of the maximum current value of the plural pulses (4 types in the example described above) as the number of types of the reference current values (4 types in the example described above). The base sequence of the polynucleotide can be determined with better precision when there are the same number of types of the plural pulse maximum current values, and types of the reference current values.

The magnitude relationship of the reference current values is determined according to the material of the electrode pairs.

For example, when the electrode pair are gold electrodes, the magnitude order of the reference current values when the nucleotides (reference nucleotides) are DNA may be dTMP < dCMP < dAMP < Methyl dAMP < dGMP < Oxo-dGMP < Methyl dCMP, and when the nucleotides (reference nucleotides) are RNA may be rUMP < rCMP < rAMP < rGMP. The present invention is obviously not limited thereto.

The third process may be configured as a process of generating primary base sequence data in which the each of the plural pulses detected from the polynucleotide is associated with a specific type of nucleotide by comparing the maximum current values measured in the second process with reference current values corresponding to individual nucleotides (referred to collectively below as reference nucleotides), or including such a process.

Namely, in the third process, each of the pulses is associated with a specific type of nucleotide by comparing a premeasured modal value of the maximum current values of the reference nucleotides (in other words reference current values) against the maximum current values of each of the pulses actually measured with the polynucleotide. The data of the maximum current values measured in the second process is thereby converted into the primary base sequence data.

Namely, in the third process, as long as there is a match between the maximum current value of the pulses actually measured using the polynucleotide and the modal value of the maximum current value of a specific reference nucleotide (in other words a reference current value) the nucleotides within the polynucleotide generating the above pulses can be determined to be the same as the above specific reference nucleotides.

The reference nucleotides referred to above mean nucleotides that possibly configure a polynucleotide. Specifically, these may be any given ribonucleotide, or any given deoxyribonucleotide, or chemical modified ribonucleotide or deoxyribonucleotide (for example subjected to methylation, oxylation, hydroxylation, formylation, carboxylation, dimerization, base-removal or the like) mentioned as nucleotides that may configure a polynucleotide under "1.1: First Process". More specific examples of nucleotides have already been explained under "1.1: First Process", and so further explanation thereof is omitted.

The above reference current values may be derived as the modal value of the maximum current values of plural pulses of tunnel current arising when a reference nucleotide is individually passed between an electrode pair. Note that the electrode pair employed when determining the reference current value preferably employs the same electrodes as in the electrode pair employed for determining the base sequence of the polynucleotide. Such a configuration enables the measurement conditions when deriving the reference current values to be made the same as the measurement conditions when determining the polynucleotide base sequence, thereby enabling the polynucleotide base sequence to be determined with high precision.

Namely, in the present invention, after fabrication of the electrode pair, first reference current values may be derived for various reference nucleotides, and data for the reference current values stored in a database. Then when actually determining the base sequence of the polynucleotide, whether or not each of the pulses arising from the polynucleotide corresponds to the reference nucleotides may be determined by comparing the maximum current value of each of the pulses arising from the polynucleotide against the reference current values in the database.

Specific explanation follows regarding a method to derive reference current values.

When deriving the reference current values, individual reference nucleotides may be passed between the electrode pair plural times, plural tunnel currents measured for each of the reference nucleotides, and a maximum current value of the plural tunnel current values derived. The most frequently occurring maximum current value may then be taken as the reference current value.

First, the reference nucleotides are individually dissolved in a solvent (for example the same solvent as that for dissolving the polynucleotide).

Then, by holding the electrode pair in the solution in which the reference nucleotide is dissolved and applying a voltage between the electrode pair, a reference nucleotide becomes trapped between the electrode pair as the reference nucleotide passes. A tunnel current arises between the electrode pair during the interval in which the reference nucleotide is trapped between the electrode pair (in the interval during which the reference nucleotide is present between the electrode pair). The reference nucleotide trapped between the electrode pair then spontaneously moves away from the electrode pair after a specific period of time has elapsed. The tunnel current arising between the electrode pair then disappears due to the reference nucleotide moving away from the electrode pair. A pulse in tunnel current accordingly arises due to the reference nucleotide being trapped between the electrode pair, and then moving away from between the electrode pair. Plural sets of tunnel current data are then obtained by repeating the trapping and moving away for each of the reference nucleotides.

There is no particular limitation to the method for applying voltage between the electrode pair, and for example a known power supply device may be connected to the electrode pair, and a voltage applied between the electrode pair (for example a bias voltage). There is no particular limitation to the voltage applied, as long as a similar voltage is employed during determination of the polynucleotide base sequence. For example 0.25V to 0.75V may be employed.

Thus the current value of the tunnel current arising between the electrode pair by applying the voltage between the electrode pair held in the solution in which the reference nucleotide is dissolved may be measured over a specific period of time. For example, the current value of the tunnel current may be measured for example for 50 minutes.

The tunnel current arising between the electrode pairs may be determined by employing a known ammeter. The tunnel current signal may moreover be first amplified employing for example a current amplifier. Since employing a current amplifier enables amplification of weak tunnel current values, it is possible to measure the tunnel current with high sensitivity. An example of a current amplifier is commercially available variable gain high speed current amplifier (Catalogue Number: DHPCA-100, manufactured by FEMTO Messtechnik GmbH).

Thus by measuring the tunnel current flowing between the electrode pair for a specific period of time, pulses of tunnel current can be detected by serially determining whether or not the current value of the tunnel current has exceeded a base level. Specifically, whilst performing the above determination, by identifying the time when the tunnel current exceeds the base level and identifying the time when the tunnel current returns once again to the base level, a tunnel current pulses caused by the reference nucleotide can be detected in the signal between these 2 times. Using a graph (for example a curved line graph) expressing the relationship between the measured current values of the tunnel current and the tunnel current measured duration enables such determination to be made easily by visual inspection.

There are various heights of peak present in the pulses caused by the thus detected reference nucleotides. These peaks appear due to changes in the distance between the electrodes and the reference nucleotide according to the movement of the reference nucleotide between the electrode pair. Namely, since tunnel current readily arises when the distance between the reference nucleotide and the electrodes is short, the current value of the tunnel current increases. However, since tunnel current does not readily arise when the distance between the reference nucleotide and the electrodes is long, the current value of the tunnel current decreases. There are accordingly changes in the distance between the electrodes and the reference nucleotide and increases and decreases in the current values of the tunnel current caused by movement of the reference nucleotide between the electrode pair, and hence plural various peaks appear in the pulse of the tunnel current.

The maximum current values of each of the pulses can accordingly be derived by subtracting the base level from the highest peak current value of each of the thus detected pulses. Then the modal value can be computed by performing statistical analysis on each of the derived maximum current values.

In order to derive the modal value, for example, a histogram is generated that expresses a relationship between the maximum current value and the number of pulses with that value. The generated histogram is fitted to a specific function. The modal value may then be calculated by deriving a peak value of the fitted function.

The function employed in fitting may be a Gaussian function or a Poisson function, and is preferably a Gaussian function. Employing a Gaussian function has the advantage of enabling the data processing speed to be made faster.

The number of samples (pulses) employed in the statistical analysis in order to calculate the modal value is not particularly limited, and is for example between 500 and 1000 individual samples. Employing a number in this region for statistical analysis enables calculation of a statistically meaningful modal value. Since such modal values are distinct values to each nucleotide, these modal values may be employed as indicators for nucleotide identification.

The inventors of the present invention demonstrate that, as illustrated in Table 1, described later, the modal values for reference nucleotides that are dGMP, dAMP, dCMP, dTMP, rGMP, rAMP, rCMP and rUMP are respectively 87pS, 67pS, 60pS, 39pS, 123pS, 92pS, 64pS, and 50pS. Moreover, the modal values for reference nucleotides that are methylcytosine, oxoguanine and ribonucleotides or deoxyribonucleotides from which the base has been removed are respectively 105pS, 98pS and 0pS (note that these modal values are calculated under conditions of an inter-electrode distance of 0.8nm, a bias voltage of 0.4V and with the number of samples set at (1000)) for statistical analysis. Since the modal values are distinct values for each reference nucleotide, these modal values may accordingly be employed as indicators for identification of nucleotides configuring the polynucleotide.

Note that the tunnel current is influenced by such factors as the inter-electrode distance, the concentration of the nucleotide or the polynucleotide in the solution, the shape of the electrodes, and the voltage between electrodes, and so the modal values calculated from the tunnel current are also influenced thereby. For example, even for the same type of nucleotide, the modal values would differ between bias voltages of 0.25V, 0.50V, and 0.75V applied between the electrodes.

There is accordingly a distribution of the above modal values. Consequently, it is possible to employ as the reference current value used in the present invention a "single point modal value", and it is also possible to employ a "modal value distribution". When a "modal value distribution" is employed as the reference current value, the "modal value distribution" may be expressed as a full width at half maximum of the function employed to derive the modal values (a Gaussian function or a Poisson function).

The reference current value employed in the present invention may be the modal value of the reference nucleotide, or may be a value in a range of $x \pm y$ wherein x is modal value of the reference nucleotide and y is the half width at half maximum of the function employed to calculate the modal value of the reference nucleotide. Moreover, since the modal value is influenced by various conditions as described above, the modal value of the reference nucleotide is preferably determined under similar conditions to the conditions during polynucleotide base sequence determination.

As illustrated in Table 1 described below, x is 87pS and y is 22pS when the reference nucleotide is dGMP. x is 67pS and y is 17ps when the reference nucleotide is dAMP. x is 60pS and y is 22pS when the reference nucleotide is dCMP. x is 39pS an y is 11pS when the reference nucleotide is dTMP. x is 123pS and y is 54pS when the reference nucleotide is rGMP. x is 92pS and y is 33pS when the reference nucleotide is rAMP. x is 64pS and y is 18ps when the reference nucleotide is rCMP. x is 50pS and y is 12pS when the reference nucleotide is rUMP.

When comparison is made between the maximum current value of each of the pulses measured in the second process and the reference current values described above, when the maximum current value falls in a range of 87pS $\pm$ 22pS, the pulse may be determined to be a pulse caused by dGMP, and when the maximum current value does not fall in the range of 87pS $\pm$ 22pS, the pulse may be determined not to be a pulse caused by dGMP.

When comparison is made between the maximum current value of each of the pulses measured in the second process and the reference current values described above, when the maximum current value falls in a range of 67pS $\pm$ 17pS, the pulse may be determined to be a pulse caused by dAMP, and when the maximum current value does not fall in the range of 67pS $\pm$ 17pS, the pulse may be determined not to be a pulse caused by dAMP.

When comparison is made between the maximum current value of each of the pulses measured in the second process and the reference current values described above, when the maximum current value falls in a range of 60pS $\pm$ 22pS, the pulse may be determined to be a pulse caused by dCMP, and when the maximum current value does not fall in the range of 60pS $\pm$ 22pS, the pulse may be determined not to be a pulse caused by dCMP.

When comparison is made between the maximum current value of each of the pulses measured in the second process and the reference current values described above, when the maximum current value falls in a range of 39pS $\pm$ 11pS, the pulse may be determined to be a pulse caused by dTMP, and when the maximum current value does not fall in the range of 39pS $\pm$ 11pS, the pulse may be determined not to be a pulse caused by dTMP.

When comparison is made between the maximum current value of each of the pulses measured in the second process and the reference current values described above, when the maximum current value falls in a range of 123pS $\pm$ 54pS, the pulse may be determined to be a pulse caused by rGMP, and when the maximum current value does not fall in the range of 123pS $\pm$ 54pS, the pulse may be determined not to be a pulse caused by rGMP.

When comparison is made between the maximum current value of each of the pulses measured in the second process and the reference current values described above, when the maximum current value falls in a range of 92pS $\pm$ 33pS, the pulse may be determined to be a pulse caused by rAMP, and when the maximum current value does not fall in the range of 92pS $\pm$ 33pS, the pulse may be determined not to be a pulse caused by rAMP.

When comparison is made between the maximum current value of each of the pulses measured in the second process and the reference current values described above, when the maximum current value falls in a range of 64pS $\pm$ 18pS, the pulse may be determined to be a pulse caused by rCMP, and when the maximum current value does not fall in the range of 64pS $\pm$ 18pS, the pulse may be determined not to be a pulse caused by rCMP.

When comparison is made between the maximum current value of each of the pulses measured in the second process and the reference current values described above, when the maximum current value falls in a range of 50pS $\pm$ 12pS, the pulse may be determined to be a pulse caused by rUMP, and when the maximum current value does not fall in the range of 50pS $\pm$ 12pS, the pulse may be determined not to be a pulse caused by rUMP.

When the maximum current value of the pulse measured in the second process belongs to plural reference nucleotide "modal value distributions", the pulse may be determined to be a pulse caused by the reference nucleotide nearest to the peak of the "modal value distribution".

As the reference current value employed in the present invention, it is possible to employ the modal values described above as they are, however it is also possible to employ a ratio in which the modal value of one or other of the nucleotides is set to "1". For example, it is possible to derive ratios of modal values of other nucleotides when the value of the modal values of dGMP or rGMP are set to "1", and to then use this ratio as the reference current value.

In such a case, for example, the ratios of the modal values of the reference nucleotides described above are dGMP : dAMP : dCMP : dTMP = 1 $\pm$ 0.25 : 0.77 $\pm$ 0.20 : 0.69 $\pm$ 0.25 : 0.45 $\pm$ 0.12, and rGMP : rAMP : rCMP : rTMP = 1 $\pm$ 0.44 : 0.75 $\pm$ 0.27 : 0.58 $\pm$ 0.16 : 0.41 $\pm$ 0.10.

In the third process, each of the pulses is associated with a specific nucleotide by determining to which of the reference current values described above the maximum current values of each of the pulses measured in the second process belong. Then, based on the associations, primary base sequence data may be generated in which each of the pulses is associated with a specific nucleotide in the sequence of time in which each of the pulses were measured in the second process.

For example, when 8 pulses are detected in the second process, these pulses may be replaced by primary base sequence data such as "AGATTCAC" according to the determination criteria described above.


1-4: Fourth Process

The fourth process is a process of extracting from out of the plural pulses a pulse group configured by pulses with successive pulse continuation durations, and of extracting from out of the primary base sequence data plural secondary base sequence data corresponding to the pulse group.

In the present specification, reference to "pulses with successive pulse continuation durations" means that the separation between pulse continuation durations of adjacent pulses is short, or in other words, that they appear to be successive pulses.

For example, when the period of time between the pulse continuation duration of a given pulse and the pulse continuation duration of a pulse adjacent to the given pulse is shorter than the pulse continuation duration corresponding to one nucleotide, then these pulses may be considered to be pulses caused by adjacent nucleotides within the polynucleotide, and classified as being in the same pulse group. Plural pulses detected in the second process are accordingly classified in plural pulse groups. Then plural sets of secondary base sequence data corresponding to each of the pulse groups are extracted from out of the primary base sequence data.

Note that there is no particular limitation to the number of pulses belonging to a single pulse group, and for example there may be any number as long as it is 2 or more. The greater the number of pulses belonging to one pulse group the longer the length of the common base sequence found in the fifth process described later. The determination precision of base sequences of the polynucleotide can be raised as a result.

For example, consider a case in which a base sequence of a polynucleotide (for example DNA or RNA) is determined to be configured by N individual bases. When N < 100, the number of pulses belonging to a single pulse group is preferably N/3 individual pulses or greater, with N/2 individual pulses more preferable, with N individual pulses being even more preferable, and with more than N individual pulses being even more preferable. When N > 100, the number of pulses belonging to a single pulse group is preferably 50 individual pulses or greater, with N/2 individual pulses more preferable, with N individual pulses being even more preferable, and with more than N individual pulses being even more preferable.

As long as there are 50 or more individual pulses belonging to a single group it is possible perform the operations of the fifth process and the sixth process with good precision.

More specifically, the number of pulses belonging to a single pulse group is preferably 3 individual pulses or greater, is more preferably 4 individual pulses or greater, is more preferably 5 individual pulses or greater, is more preferably 6 individual pulses or greater and is more preferably 7 individual pulses or greater. The greater the number of pulses belonging to a single group the better.

The "pulse continuation duration corresponding to one nucleotide" described above may, for example, be determined by deriving the pulse continuation duration of the reference nucleotide at the same time as when measuring the modal value of the maximum current values of a reference nucleotide.

For example, when the pulse continuation duration is measured for various types of reference nucleotide, it is possible to take the pulse continuation duration of one of these pulse continuation durations (for example the shortest pulse continuation duration) as the "pulse continuation duration corresponding to one nucleotide" described above.

For example, as illustrated in the exemplary embodiments, the modal value of the pulse continuation duration of dGMP is about 0.8ms (note that that these modal values are calculated under conditions of an inter-electrode distance of 0.8nm, a bias voltage of 0.4V and with the number of samples for statistical analysis set at (1000)). As long as the period of time between the pulse continuation duration of one pulse and the pulse continuation duration of a pulse adjacent to this pulse is shorter than 0.8ms, then these pulses may be considered to be pulses caused by adjacent nucleotides within the polynucleotide.

Moreover, in the fourth process, the plural secondary base sequence data corresponding to a pulse group configured by pulses with successive pulse continuation durations is preferably extracted over a period of time of 1ms or greater.

As described above, the pulse continuation duration of pulses corresponding to one nucleotide is preferably about 0.8ms to about 1ms. As a result, when the pulse continuation duration continues for a period of time of at least 1ms or greater, then noise when measuring the tunnel current can be eliminated, thereby enabling the polynucleotide base sequence to be determined with better precision.

Moreover, longer the total period of time over which the pulse continuation duration continues the better. For example, preferably the pulse continuation duration continues for a period of time of at least 2ms or greater, at least 5ms or greater, or at least 10ms or greater. Adopting such a configuration not only enables noise to be excluded, but also enables a longer polynucleotide base sequence to be determined.

In the fourth process, the secondary base sequence is preferably extracted based on a probabilistic statistical method (for example probabilistic theory based on a Gaussian function or a Poisson function). In other words, in the fourth process, preferably the secondary base sequence data configured by nucleotides with the highest appearance probability out of plural nucleotide candidates obtained that correspond to the measured tunnel current are extracted according to the probabilistic statistical method.

As illustrated in Fig. 2(c) and (d), there is a distribution to the tunnel current caused by each of the nucleotides, and there are portions of each of the distributions that overlap with each other. The reason for this is that when specific values of the tunnel current are measured, there are plural candidates of nucleotides for generating that tunnel current. However, since there is not a match between the whole distribution of the tunnel currents cause by each of the nucleotides, when the specific value of the tunnel current is measured the likelihood (appearance probability) differs between each of the nucleotide candidates for generated the tunnel current (see, for example, Fig. 2(c) and (d)).

For example, as the probabilistic statistical method referred to above, a method may be employed in which the measured tunnel current and the base molecule with the highest appearance probability in relation to that tunnel current are associated with each other.

For example, consider a case when the base molecule is "A", "T", "G" or "C". When the tunnel current has been measured, then the following relationship equation stands between P(A) denoting the probability that the substance flowing the tunnel current is "A", P(T) denoting the probability that the substance flowing the tunnel current is "T", P(G) denoting the probability that the substance flowing the tunnel current is "G", and P(C) denoting the probability that the substance flowing the tunnel current is "C". Namely,

$$1 = P(A) + P(T) + P(G) + P(C).$$

In such a case P(X) with the highest value (wherein X is A, T, G or C) may be associated with the tunnel current. Namely, the tunnel current may be determined as being caused by the base molecule X.

For example, in a case in which when the specific values of the tunnel currents are measured, the P(A) is 35%, the P(G) is 50%, the P(C) is 10% and the P(T) is 5%, the tunnel current may be determined to be caused by "G" that is the nucleotide with the highest probability.

The secondary base sequence data may be thought of as data determined under substantially the same conditions corresponding to a pulse group configured from plural successive pulses. By comparing the plural successive pulses against each other, a secondary base sequence is extracted based on a probabilistic statistical method, enabling a more accurate secondary base sequence to be extracted.

Moreover, when the secondary base sequence is extracted based on a probabilistic statistical method, it is even more preferable to employ an electrode pair in which the inter-electrode distance is maintained constant. Namely, the above electrode pair is preferably an electrode pair in which the inter-electrode distance does not change during the time the tunnel current is being measured.

For example, preferably the proportional change in the inter-electrode distance is preferably 1% or less, more preferably 0.1 % or less, even more preferably 0.01% or less, and even more preferably 0.001% or less.

Electrode pairs fabricated by conventional technology seem at first glance to maintain a constant inter-electrode distance when observed with the naked eye, however there are actually microscopic changes in the inter-electrode distance. When there are even microscopic changes in the inter-electrode distance this leads to fluctuations in the tunnel current values. Namely, tunnel current values caused by the same substance vary, lowering the determination precision of polynucleotide base sequences.

Namely, with electrode pairs fabricated by conventional technology, the distributions illustrated in Fig. 2(c) and (d) readily fluctuate, with accompanying difficulties in identifying the type of the nucleotide.

However, by employing an electrode pair capable of maintaining a constant inter-electrode distance, the distributions illustrated in Fig. 2(c) and (d) can be maintained, thereby enabling the determination precision of the polynucleotide base sequence to be raised even higher.

Namely, employing an electrode pair capable of maintaining a constant inter-electrode distance enables a stable tunnel current to be measured that is not influenced by the measurement environment. As a result this enables the determination precision of the polynucleotide base sequence to be raised even higher.

There are large fluctuations in the tunnel current due to various parameters. For example, there are large fluctuations due to inter-electrode distance. This is thought to be the reason that those of ordinary skill in the art have not considered the possibility of determining a polynucleotide base sequence based on tunnel current. However, as shown in the exemplary embodiment, the present inventors have demonstrated that polynucleotide base sequence can be determined based on the tunnel current.

Then, employing the probabilistic statistical method in the fourth process and employing an electrode pair capable of maintaining a constant inter-electrode distance enables the polynucleotide base sequence to be determined with even higher precision.

Imperfections in the precision of the secondary base sequence readily occur in cases not employing the above configuration. There are also occasions when the precision of secondary base sequences is lowered in such cases (for example about 10% or lower).

However, the above configuration enables measurements of the tunnel current to be stabilized further, and so the precision of secondary base sequence data extracted by a probabilistic statistical method can be raised. Namely, more accurate secondary base sequence extraction is enabled. As a result, more accurate determination of polynucleotide base sequences based on data relating to tunnel current is enabled. More specifically, the above configuration enables secondary base sequence extraction at a stable precision of about 80% or higher, enabling accurate determination of polynucleotide base sequences based on high precision secondary base sequences.

For example, tunnel current measurements are conventionally performed employing a Scanning Tunnelling Microscope (STM) that has an extremely large configuration, however it is fundamentally not easy to make tunnel current measurements in a solution using an STM, and it is difficult to maintain a constant inter-electrode distance. However, in a feedback method using a piezoelectric actuator, with a nano-gap electrode mechanically-controllable break junction made from a fine metallic line fabricated by nanofabrication, or a nano-gap electrode fabricated by nanofabrication on a substrate, it is easy to maintain a constant inter-electrode distance even in a solution.

1-5: Fifth Process

The fifth process is a process of searching the plural secondary base sequence data for base sequences that are common across at least two of the secondary base sequence data. Namely, the fifth process is a process that searches the secondary base sequence data that is fragmented full length base sequence data of a polynucleotide for locations where secondary base sequence data are joined together.

There is no particular limitation to the length of common base sequences, and it may be a length of 2 or more bases, may be a length of 3 or more bases, may be a length of 4 or more bases, may be a length of 5 or more bases, or may be length of 10 or more bases. In order to determine a polynucleotide base sequence with good precision, the length of the common base sequence is preferably as long as possible.

The common base sequences searched for in the fifth process are preferably common across as many instances of secondary base sequence data as possible. For example, it is preferable to be common across at least 2 secondary base sequence data, more preferably common across at least 5 secondary base sequence data, more preferably common across at least 10 secondary base sequence data, more preferably common across at least 15 secondary base sequence data, and more preferably common across at least 20 secondary base sequence data. The above configuration enables a polynucleotide base sequence to be determined with better precision.

1-6: Sixth Process

The sixth process is a process of joining the secondary base sequence data that have the common base sequence together through the common base sequence. A base sequence for a polynucleotide (full length or part length) can be determined according to this process.

In a case in which in the fifth process "AGATT", "GATTC" and "TTCAC" are obtained as secondary base sequence data having common base sequences, "AGATTC" is for example obtained by joining together "AGATT" and "GATTC" through "GATT". Then "AGATTCAC" is obtained by joining the "AGATTC" and the "TTCAC" together through the "TTC".

The above configuration enables a longer polynucleotide base sequence to be determined.

In the sixth process, configuration may be made such that plural sequence data of base sequences found to be common at the fifth process are extracted as tertiary base sequence data, and the tertiary base sequence data are then joined together.

For example, in a case in which in the fifth process "AGATT", "GATTC" and "TTCAC" are obtained as secondary base sequence data having common base sequences, "GATT" that is common to "AGATT" and "GATTC", and "TTC" that is common to "GATTC" and "TTCAC" are extracted as tertiary base sequence data. Then "GATTC" is obtained by joining together "GATT" and "TTC" through "TT".

The above configuration enables a polynucleotide base sequence to be determined with better precision. Namely, since the section "GATTC" appears plural times in the base sequence being identified, it may be said to be a sequence that has an extremely high reliability.

2. Polynucleotide Base Sequence Determination Device

2.1: Regarding Each Configuration

A polynucleotide base sequence determination device of the present exemplary embodiment is a device for executing the polynucleotide base sequence determination method of the present invention.

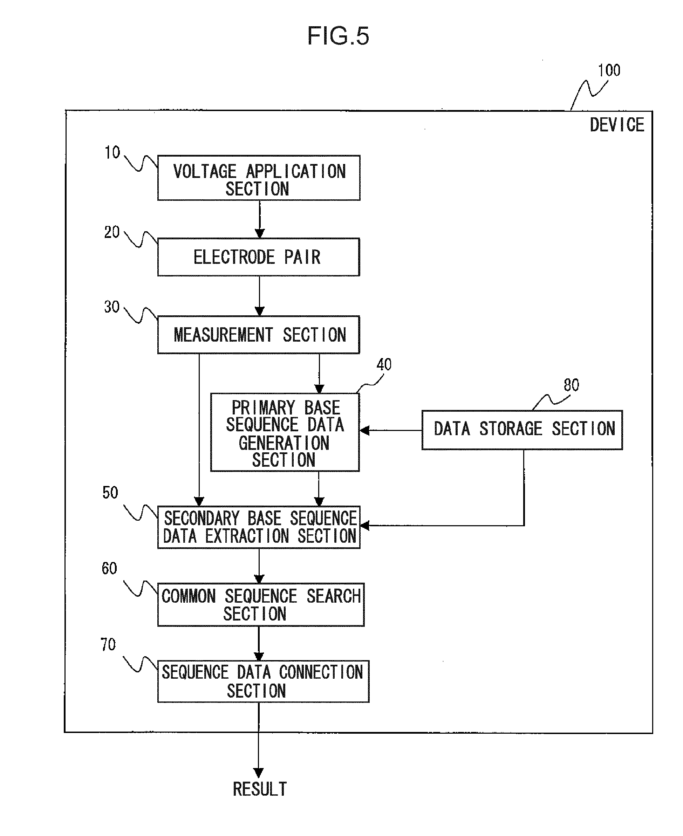

Explanation follows regarding a configuration of such a device, with reference to Fig. 5, however the configuration illustrated in Fig. 5 is merely an example, and the present invention is not limited thereto. Note that matter already explained in the section "1. Polynucleotide Base Sequence Determination Method" is omitted from further explanation.

A device 100 of the present exemplary embodiment includes a voltage application section 10, an electrode pair 20, a measurement section 30, a primary base sequence data generation section 40, a secondary base sequence data extraction section 50, a common sequence search section 60, a sequence data connection section 70 and a data storage section 80. Explanation follows regarding configuration of each.

The voltage application section 10 is configured to apply a voltage to the electrode pair 20.

The magnitude of the voltage applied by the voltage application section 10 to the electrode pair 20 is not particularly limited, and may for example be 0.25V to 0.75V.

There is no particular limitation to the specific configuration of the voltage application section 10, and it is possible to employ a suitable known voltage application device.

The polynucleotide moves between the electrode pair 20 on application of the voltage by the voltage application section 10, and when this occurs a tunnel current arises between the electrode pair 20. Then in the present invention, a polynucleotide base sequence is determined based on the tunnel current.

Since detailed explanation has already been given of a specific configuration of the electrode pair 20 further explanation thereof is omitted.

The measurement section 30 detects plural pulses of tunnel current arising as the polynucleotide passes between the electrode pair 20, and is configured to measure the maximum current value and the pulse continuation duration for each of the plural pulses. There is no particular limitation to the specific configuration of the measurement section 30, and a suitable, known ammeter device may be employed therefor.

Out of the data determined by the measurement section 30, at least data relating to the maximum current value is transmitted to the primary base sequence data generation section 40. Moreover, data compiled in the data storage section 80 relating to reference current values of the reference nucleotides is also transmitted to the primary base sequence data generation section 40.

In the primary base sequence data generation section 40, primary base sequence data in which each of the plural pulses detected by the measurement section 30 is associated with a specific type of nucleotide is generated by comparing the magnitude order between the maximum current values of the plural pulses and a magnitude order between reference current values.

In the primary base sequence data generation section 40, the primary base sequence data in which each of the plural pulses detected by the measurement section 30 is associated with a specific type of nucleotide may be generated by comparing data related to maximum current values measured by the measurement section 30 against data compiled in the data storage section 80 relating to the reference current values.

There is no limitation to the specific configuration of the primary base sequence data generation section 40 and the data storage section 80, and a known computing device such as a computer and a memory may be employed therefor.

The secondary base sequence data extraction section 50 at least receives data relating to the pulse continuation duration sent from the measurement section 30, the primary base sequence data sent from the primary base sequence data generation section 40, and the reference nucleotide pulse continuation duration sent from the data storage section 80. Based on these data, the secondary base sequence data extraction section 50 then extracts from out of the plural pulses a pulse group configured by pulses with successive pulse continuation durations, and extracts from out of the primary base sequence data plural secondary base sequence data corresponding to the pulse group.

Note that the secondary base sequence data extraction section 50 may be configured to extract plural secondary base sequence data corresponding to pulse groups configured by pulses with successive pulse continuation durations over an interval of time of 1ms or greater.

There is no limitation to the specific configuration of the secondary base sequence data extraction section 50, and a known computing device such as a computer may be employed therefor.

The common sequence search section 60 searches the plural secondary base sequence data received from the secondary base sequence data extraction section 50 for base sequences that are common across at least two of the secondary base sequence data out of the plural secondary base sequence data.

Note that the common base sequences searched for by the common sequence search section 60 may be configured to be common across at least 10 individual instances of secondary base sequence data.

There is no particular limitation to the specific configuration of the common sequence search section 60, and a known computing device such as a computer may be employed therefor.

The sequence data connection section 70 receives data relating to common base sequences from the common sequence search section 60 and joins together the secondary base sequence data that with a common base sequence through the common base sequence.

Note that the sequence data connection section 70 may be configured to extract as tertiary base sequence data sequence data plural base sequences found to be common by the common sequence search section 60, and join together the tertiary base sequence data.

The data connected together by the sequence data connection section 70 is a detection result.

There is no particular limitation to the specific configuration of the sequence data connection section 70, and a known computing device such as a computer may be employed therefor.


2-2: Example of Operation Flow of Device 100

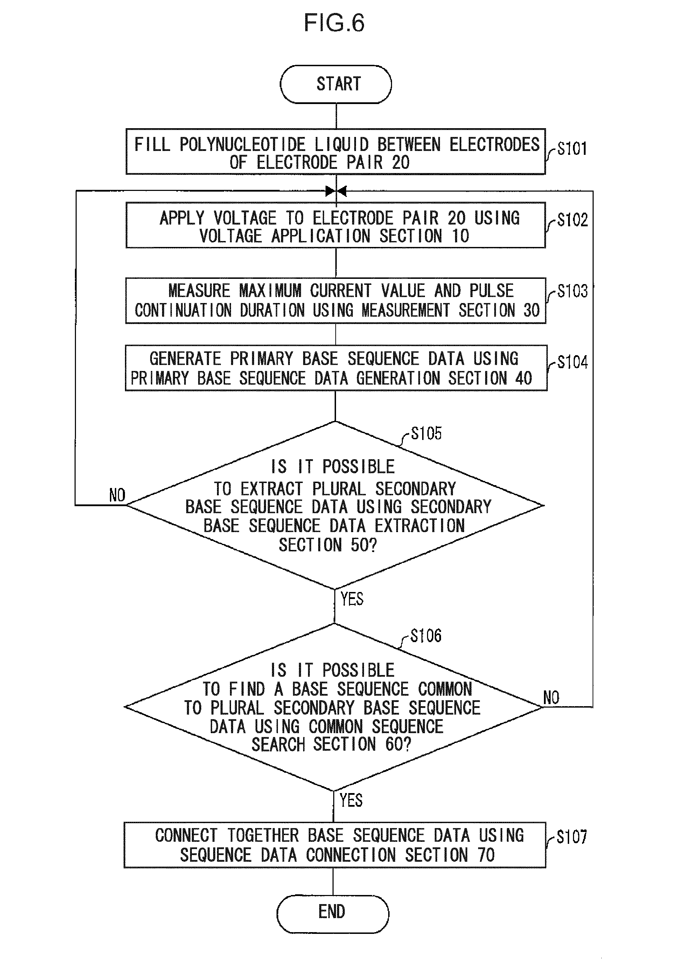

Fig. 6 illustrates an example of an operation flow of the device 100. Note that the flow is merely an example, and the present invention is not limited thereto.

At S101, a solution including a polynucleotide is filled between the electrode pair 20.

At S102, a voltage is applied to the electrode pair 20 by the voltage application section 10. A tunnel current accordingly flows through the polynucleotide present between the electrode pair 20.

At S103, the measurement section 30 detects plural pulses of tunnel current, and measures for each of the plural pulses detected a maximum current value and a pulse continuation duration.

At S104, primary base sequence data is generated for the primary base sequence data generation section 40 based on the above maximum current value and the like.

At S105, extraction of plural secondary base sequence data is performed by the secondary base sequence data extraction section 50. Processing returns to S102 when plural secondary base sequence data could not be extracted when this is performed. Processing proceeds to S 106 when plural secondary base sequence data could be extracted when this is performed.

At S106, a search is performed by the common sequence search section 60 for base sequences that are common to plural instances of secondary base sequence data. Processing returns to S102 when base sequences common to plural instances of secondary base sequence data could not be found when this is performed. Processing proceeds to S 107 when base sequences common to plural instances of secondary base sequence data could be found when this is performed.

At S107, the base sequence data is connected together by the sequence data connection section 70.

In the device 100 of the exemplary embodiment described above, each part of the configuration of the device 100 and each step may be implemented by a computation means such as a CPU executing a program stored in a storage means such as Read Only Memory (ROM) or RAM, and controlled by an input means such as a keyboard, an output means such as a display, or a communication means such as an interface circuit.

Consequently, it is possible to implement the device 100 described above, each of the parts of the configuration of the device 100, and each of the steps by a computer including each means simply reading a recording medium stored with the program described above and executing the program. Moreover, various functions and various processing may be implemented by any given computer by storing the above program on a removable recording medium.

The recording medium described above may be configured by a program medium, such as a memory such as ROM, not illustrated in the drawings, that performs processing in a microcomputer, or by providing a program reading device such as an external storage device, and using a readable program medium by inserting a recording medium therein.

Moreover, in any of these cases, preferably configuration is made such that a microprocessor accesses and executes the stored program. Moreover, preferably a method is employed in which the program is read, and the read program is downloaded into a program storage area of a microcomputer, and the program then executed. Note that the downloadable program is preferably pre-stored on a main device.

The above program medium may be a storage medium configured separable to the main body, and may be a tape system such as a magnetic tape or cassette tape; a disk system such as a magnetic disk, for example a flexible disk or hard disk, or a CD/MO/MD/DVD disk; a card system such as an IC card (including memory cards), or a non-volatile storage medium holding the program, including semiconductor memory, such as a mask ROM, an Erasable Programmable Read Only Memory (EPROM), an Electrically Erasable Programmable Read Only Memory (EEPROM), or a flash ROM.

Moreover, in a system configuration that is connectable to a communication network, including the internet, preferably a storage medium is employed that dynamically holds the program so as to download the program from a communication network.

When the program is thus downloaded from a communication network, the downloadable program may be pre-stored on the main device or may be installed from a separate storage medium.

The present invention may be also configured as described below.

In order to solve the above problem, a polynucleotide base sequence determination method of the present invention includes: a first process of passing the polynucleotide between an electrode pair; a second process of detecting plural pulses of tunnel current arising as the polynucleotide passes through between the electrode pair, and of measuring the maximum current value and pulse continuation duration for each of the plural pulses; a third process of generating primary base sequence data in which each of the plural pulses is associated with a specific type of nucleotide by comparing a magnitude order between the maximum current values of the plural pulses and a magnitude order between reference current values corresponding to electron states caused by energy level differences between individual nucleotides and metal configuring the electrode pair; a fourth process of extracting from out of the plural pulses a pulse group configured by pulses with successive pulse continuation durations, and of extracting from out of the primary base sequence data plural secondary base sequence data corresponding to the pulse group; a fifth process of searching the plural secondary base sequence data for base sequences that are common across at least two of the secondary base sequence data; and a sixth process of joining the secondary base sequence data that have the common base sequence together through the common base sequence.

The above configuration enables a polynucleotide base sequence to be determined based on data relating to tunnel current.

In the polynucleotide base sequence determination method of the present invention, preferably the third process further includes generating the primary base sequence data in which each of the plural pulses are associated with a specific type of nucleotide by comparing the maximum current value against reference current values corresponding to individual nucleotides.

The above configuration enables a polynucleotide base sequence to be determined based on data relating to tunnel current.

In the polynucleotide base sequence determination method of the present invention, preferably the reference current values are modal values out of the maximum current values of the plural pulses of tunnel current arising when the nucleotide is individually passed between the electrode pair.

The above configuration enables optimization of the values of the reference current value, enabling a polynucleotide base sequence to be determined more precisely.

In the polynucleotide base sequence determination method of the present invention, preferably the electrode pair are gold electrodes; and the magnitude order of the reference current values when the nucleotides are DNA is dTMP < dCMP < dAMP < Methyl dAMP < dGMP < Oxo-dGMP < Methyl dCMP, and the magnitude order of the reference current values when the nucleotides are RNA is rUMP < rCMP < rAMP < rGMP.

The above configuration enables a polynucleotide base sequence to be determined based on data relating to tunnel current.

In the polynucleotide base sequence determination method of the present invention, preferably the sixth process extracts as tertiary base sequence data plural sequence data of a base sequence found to be common in the fifth process, and joins together the tertiary base sequence data.

The above configuration determines the base sequence using only the high reliability tertiary base sequence data, thereby enabling the polynucleotide base sequence to be determined more precisely.

In the polynucleotide base sequence determination method of the present invention, preferably the common base sequence found at the fifth process is a sequence that is common across at least 10 individual secondary base sequence data.

The above configuration determines the base sequence using more reliable common base sequences, thereby enabling the polynucleotide base sequence to be determined more precisely.

In the polynucleotide base sequence determination method of the present invention, preferably in the fourth process, plural secondary base sequence data are extracted that correspond to a pulse group configured from pulses with successive pulse continuation durations over a period of time of 1ms or longer.

According to the above configuration, not only can noise be excluded, but longer secondary base sequence data can also be obtained, thereby enabling polynucleotide base sequence determination to be made more efficiently.

In the polynucleotide base sequence determination method of the present invention, preferably the electrode pair is an electrode pair in which the inter-electrode distance is maintained constant, and in the fourth process, the secondary base sequence data is extracted using a probabilistic statistical method.

Imperfections in the precision of the secondary base sequence readily occur in cases not employing the above configuration. There are also occasions when the precision of secondary base sequences is lowered in such cases (for example about 10% or lower).

However, the above configuration enables measurements of the tunnel current to be stabilized further, and so the precision of secondary base sequence data extracted by a probabilistic statistical method can be raised. Namely, more accurate secondary base sequence extraction is enabled. As a result, more accurate determination of polynucleotide base sequences based on data relating to tunnel current is enabled. More specifically, the above configuration enables secondary base sequence extraction at a stable precision of about 80% or higher, enabling accurate determination of polynucleotide base sequences based on high precision secondary base sequences.

In order to solve the above problem, a polynucleotide base sequence determination device of the present invention includes: an electrode pair that has an inter-electrode distance through which a polynucleotide is capable of passing; a measurement section that detects plural pulses of tunnel current arising as the polynucleotide passes through between the electrode pair, and that measures the maximum current value and pulse continuation duration for each of the plural pulses; a primary base sequence data generating section that generates primary base sequence data in which each of the plural pulses is associated with a specific type of nucleotide by comparing a magnitude order between the maximum current values of the plural pulses and a magnitude order between reference current values corresponding to electron states caused by energy level differences between individual nucleotides and metal configuring the electrode pair; a secondary base sequence data extraction section that extracts from out of the plural pulses a pulse group configured by pulses with successive pulse continuation durations, and that extracts from out of the primary base sequence data plural secondary base sequence data corresponding to the pulse group; a common sequence search section that searches the plural secondary base sequence data for base sequences that are common across at least two of the secondary base sequence data; and a sequence data connection section that joins the secondary base sequence data that have the common base sequence together through the common base sequence.

The above configuration enables a polynucleotide base sequence to be determined based on data relating to tunnel current.

In the polynucleotide base sequence determination device of the present invention, preferably the primary base sequence data generating section further generates the primary base sequence data in which each of the plural pulses are associated with a specific type of nucleotide by comparing the maximum current value against reference current values corresponding to individual nucleotides.

The above configuration enables a polynucleotide base sequence to be determined based on data relating to tunnel current.

In the polynucleotide base sequence determination device of the present invention, preferably the reference current values are modal values out of the maximum current values of the plural pulses of tunnel current arising when the nucleotide is individually passed between the electrode pair.

The above configuration enables optimization of the values of the reference current value, enabling a polynucleotide base sequence to be determined more precisely.

In the polynucleotide base sequence determination device of the present invention, preferably the electrode pair are gold electrodes; and the magnitude order of the reference current values when the nucleotides are DNA is dTMP < dCMP < dAMP < Methyl dAMP < dGMP < Oxo-dGMP < Methyl dCMP, and the magnitude order of the reference current values when the nucleotides are RNA is rUMP < rCMP < rAMP < rGMP.

The above configuration enables a polynucleotide base sequence to be determined based on data relating to tunnel current.

In the polynucleotide base sequence determination device of the present invention, preferably the sequence data connection section extracts as tertiary base sequence data of plural sequence data of a base sequence found to be common by the common sequence search section, and joins together the tertiary base sequence data.

The above configuration determines the base sequence using only the more reliable tertiary base sequence data, thereby enabling the polynucleotide base sequence to be determined more precisely.

In the polynucleotide base sequence determination device of the present invention, preferably the common base sequence found by the common sequence search section is a sequence that is common across at least 10 individual secondary base sequence data.

The above configuration determines the base sequence using high reliability common base sequences, thereby enabling the polynucleotide base sequence to be determined more precisely.

In the polynucleotide base sequence determination device of the present invention, preferably the secondary base sequence data extraction section extracts plural secondary base sequence data that correspond to a pulse group configured from pulses with successive pulse continuation durations over a period of time of 1ms or longer.

According to the above configuration, since not only can noise be excluded, but longer secondary base sequence data can also be obtained, polynucleotide base sequence determination can accordingly be made more efficiently.

In the polynucleotide base sequence determination device of the present invention, preferably the electrode pair is an electrode pair in which the inter-electrode distance is maintained constant, and the secondary base sequence data extraction section extracts the secondary base sequence data using a probabilistic statistical method.

Imperfections in the precision of the secondary base sequence readily occur in cases not employing the above configuration. There are also occasions when the precision of secondary base sequences is lowered in such cases (for example about 10% or lower).

However, the above configuration enables measurements of the tunnel current to be stabilized further, and so the precision of secondary base sequence data extracted by a probabilistic statistical method can be raised. Namely, more accurate secondary base sequence extraction is enabled. As a result, more accurate determination of polynucleotide base sequences based on data relating to tunnel current is enabled. More specifically, the above configuration enables secondary base sequence extraction at a stable precision of about 80% or higher, enabling accurate determination of polynucleotide base sequences based on high precision secondary base sequences.

The present invention may be configured as described below. Obviously various combinations are possible of the following configuration and other possible configurations described in the present specification.

In order to solve the above problem, a polynucleotide base sequence determination method of the present invention includes: a first process of passing the polynucleotide between an electrode pair; a second process of detecting plural pulses of tunnel current arising as the polynucleotide passes through between the electrode pair, and of measuring the maximum current value and pulse continuation duration for each of the plural pulses; a third process of generating primary base sequence data in which each of the plural pulses is associated with a specific type of nucleotide by comparing the maximum current values against reference current values corresponding to individual nucleotides; a fourth process of extracting from out of the plural pulses a pulse group configured by pulses with successive pulse continuation durations, and of extracting from out of the primary base sequence data plural secondary base sequence data corresponding to the pulse group; a fifth process of searching the plural secondary base sequence data for base sequences that are common across at least two of the secondary base sequence data; and a sixth process of joining the secondary base sequence data that have the common base sequence together through the common base sequence.

The above configuration enables a polynucleotide base sequence to be determined based on data relating to tunnel current.

In order to solve the above problem, a polynucleotide base sequence determination device of the present invention includes: an electrode pair that has an inter-electrode distance through which a polynucleotide is capable of passing; a measurement section that detects plural pulses of tunnel current arising as the polynucleotide passes through between the electrode pair, and that measures the maximum current value and pulse continuation duration for each of the plural pulses; a primary base sequence data generating section that generates primary base sequence data in which each of the plural pulses is associated with a specific type of nucleotide by comparing the maximum current values and reference current values corresponding to individual nucleotides; a secondary base sequence data extraction section that extracts from out of the plural pulses a pulse group configured by pulses with successive pulse continuation durations, and that extracts from out of the primary base sequence data plural secondary base sequence data corresponding to the pulse group; a common sequence search section that searches the plural secondary base sequence data for base sequences that are common across at least two of the secondary base sequence data; and a sequence data connection section that joins the secondary base sequence data that have the common base sequence together through the common base sequence.

The above configuration enables a polynucleotide base sequence to be determined based on data relating to tunnel current.

Examples

1. Electrode Pair Fabrication

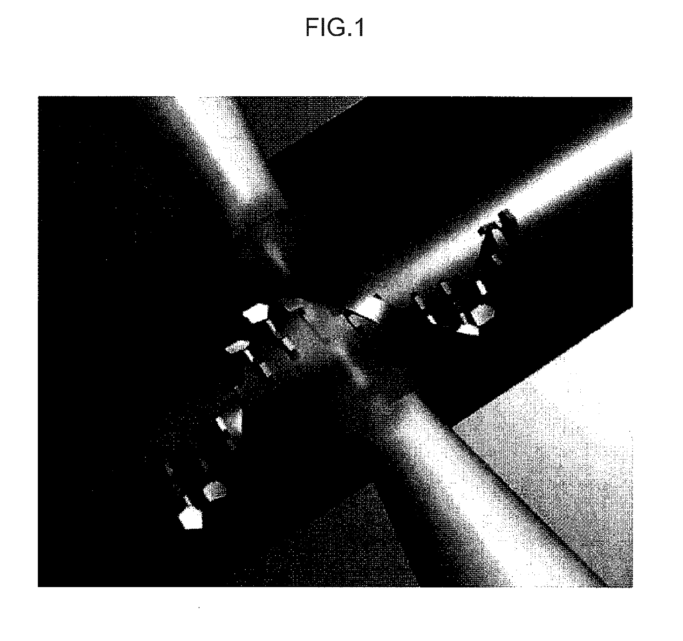

The electrode pair illustrated in Fig. 1 are formed using a nanofabricated mechanically-controllable break junction (MCBJ) method (see Tsutsui, M., Shoji, K., Taniguchi, M., Kawai, T., Formation and self-breaking mechanism of stable atom-sized junctions. Nano Lett. 8, 345-349 (2007). A simple explanation follows regarding an electrode pair fabrication method.

A nanoscale gold junction is formed as a pattern on a polyimide (Catalogue Number: Pyre-M1, manufactured by Industrial Summit Technology) coated flexible metal substrate (phosphor-bronze substrate) using an electron beam lithography device (Catalogue number: JSM6500F, manufactured by JEOL Ltd.,) and standard electron beam lithography and lift-off technology.

Next, polyimide beneath the junction is removed by etching based on a reactive ion etching process employing a reactive ion etching device (catalogue number: 10NR, made by Samco Inc.,). A nanoscale gold bridge with a 3-point bent structure is then fabricated by bending the metal substrate. Note that such bending of the substrate is performed by employing a piezoelectric actuator (catalogue number: APA150M, made by CEDRAT).

Next, the above bridge is pulled, and the electrode pair (gold electrodes) are formed by breaking a portion of the bridge. Specifically, a data acquisition board (made by National Instruments Corporation, catalogue number: NI PCIe-6321) is employed to apply a DC bias voltage (Vb) of 0.1 V to the bridge employing series resistance of $10k\Omega$ at a programmed junction stretching speed, pulling the bridge, and breaking the bridge by a resistance feedback method. Next, the bridge is pulled further and the size of the gap (inter-electrode distance) occurring due to the break is set to the length of the target nucleotide molecule (about 1nm).

The electrode pair is obtained by the above sequence. Note that observation of the fabricated electrode pair using a microscope reveals that the inter-electrode distance of the electrode pair is 0.08nm.

2. Measurement of the tunnel current arising between the electrode pair

The electrode pair is immersed in Milli-Q water in which a nucleotide or polynucleotide is dissolved, and tunnel current arising when the nucleotide or polynucleotide is trapped between the electrode pair is measured. Note that the concentrations of the nucleotide or polynucleotide in the Milli-Q water are both $0.10\mu$M.

Measurements are performed at 10kHz of the tunnel current flowing between the electrode pair with inter-electrode distance of 0.80nm length under DC bias voltage of 0.4V using a logarithmic amplifier (made by Daiwa Giken (Ltd.) according to design described in Rev. Sci. Instrum. 68 (10), 3816, and a PX1 4071 digital multimeter (National Instruments). Measurement was performed until 200 individual or 1000 individual pulses had been detected for each sample, and analysis performed of these pulses.

3. Reference Nucleotide Maximum Current Value and Pulse Continuation Duration Measurement

4 types of deoxyribonucleoside monophosphates (dAMP 2'- deoxyadenosine-5' -monophosphate: Sigma-Aldrich), dCMP (2'-deoxycytidine-5'-monophosphate sodium salt: Sigma-Aldrich), dGMP (2'-deoxyguanosine-5'-monophosphate sodium salt hydrate: Sigma-Aldrich), dTMP (Thymidylic acid disodium salt: Tokyo Chemical Industry Co. (TCI))) and 4 types of ribonucleoside monophosphates (rAMP (2'-adenosine-5'-monophosphate disodium salt: Oriental yeast), rCMP (cytidine 5'-monophosphate disodium salt: TCI), rGMP (Guanosine 5'-monophosphate sodium salt hydrate: TCI), rUMP (uridine 5'-monophosphate disodium salt hydrate: TCI)) are individually passed between the above electrode pair, and the tunnel currents arising between the electrode pair at that time are measured, and the maximum current value and the pulse continuation duration are measured for the pulses of tunnel current. As separate samples, measurements are also taken for methylcytosine, methyladenine, and oxoguanine.

Specifically, measurement solutions are produced by adding each of the deoxyribonucleoside monophosphates or the ribonucleoside monophosphates to Milli-Q water until a final concentration of $0.10\mu$M is reached.

In a state in which the measurement solution is filled in the space between the electrodes, a voltage of 0.4V is applied between the nano-gap electrodes, and the tunnel current arising between the electrodes measured. Note that when this is performed, the deoxyribonucleoside monophosphate or the ribonucleoside monophosphate between the electrodes moves by Brownian motion (the temperature of the measurement solution is about 25°C).

Fig. 2 (a) illustrates data when a measurement solution containing dGMP is employed and tunnel current is measured over the passage of time. As illustrated in Fig. 2(a), plural pulses of tunnel current are observed over the passage of time. The magnitude of the tunnel current is about 10pA to about 100pA.

Fig. 2 (b) illustrates an example of one of the plural tunnel current pulses illustrated in Fig. 2 (a). As illustrated in Fig. 2 (b), it is possible to measure Ip (maximum current value) and td (pulse continuation duration) for each of the pulses. For example, typical Ip and td for dGMP are Ip = 100pA, td = 1ms.

About 1000 individual pulses are used for each of the deoxyribonucleoside monophosphates and each of the ribonucleoside monophosphates to produce a conductance (Ip/V) histogram. Note that a Gaussian distribution is employed to produce the conductance (Ip/V) histogram.

Fig. 2 (c) and Fig. 2 (d) illustrate conductance (Ip/V) histograms. As illustrated in Fig. 2 (c) and Fig. 2 (d), the G values (G value = the peak value of the conductance (Ip/V) histogram) of each of the nucleic acid monomers are 87pS for dGMP, 67pS for dAMP, 60pS for dCMP, 39pS for dTMP, 123pS for rGMP, 92pS for rAMP, 64pS for rCMP, and 50pS for rUMP. Comparing the magnitudes of these values gives dGMP (87pS) > dAMP (67pS) > dCMP (60pS) > dTMP (39pS) for DNA, and gives rGMP (123pS) > rAMP (92pS) > rCMP(64pS) > rUMP (50pS) for RNA.

Standardized values for G values of dGMP or rGMP are illustrated in Table 1.

| Type of nucleic acid monomer | G value (pS) | Relative G value $\pm$ FWHM |
|---|---|---|
| dGMP | 87 | 1.00 $\pm$ 0.25 |
| dAMP | 67 | 0.77 $\pm$ 0.20 |
| dCMP | 60 | 0.69 $\pm$ 0.25 |
| dTMP | 39 | 0.45 $\pm$ 0.12 |
| rGMP | 123 | 1.00 $\pm$ 0.44 |
| rAMP | 92 | 0.75 $\pm$ 0.27 |

(continued)

| Type of nucleic acid monomer | G value (pS) | Relative G value ± FWHM |
|---|---|---|
| rCMP | 64 | 0.58 ± 0.16 |
| rUMP | 50 | 0.41 ± 0.10 |
| FWHM: Full Width at Half Maximum | | |

According to calculation based on density functional theory, the highest energy occupied molecular orbit (HOMO) are -5.7eV for guanine, -5.9eV for adenine, -6.1eV for cytosine, -6.6eV for thymine and -6.9eV for uracil. Comparing the magnitude of the numerical values gives: guanine (-5.7 eV) > adenine (-5.9 eV) > cytosine (-6.1 eV) > thymine (-6.6 eV) > uracil (-6.9 eV).

The magnitude order of the highest energy occupied molecular orbits is the same as the order of the relative G value magnitudes described above. This illustrates a method to identify types of molecule based on the energy levels (in particular, HOMO energy levels) by determining the tunnel currents of the types of nucleic acid monomer.

Moreover, it is clear that the type of nucleic acid monomer can be identified by employing the "relative G value ± FWHM"

Note that although not illustrated in Table 1, the G values of each of methylcytosine and oxoguanine are 105pS, 98pS.

Separate test results measured under the same test sequence are illustrated in Table 2.

The test results of Table 1 and the test results of Table 2 illustrate the same tendency.

| Type of nucleic acid monomer | G value (pS) | Relative G value ± FWHM |
|---|---|---|
| Oxo-dGMP | 81.4 | 1.13 ± 0.31 |
| Methyl dCMP | 87.2 | 1.21 ± 0.27 |
| dGMP | 72.1 | 1.00 ± 0.25 |
| Methyl dAMP | 63.3 | 0.88 ± 0.21 |
| dAMP | 51.9 | 0.77 ± 0.20 |
| dCMP | 49.7 | 0.69 ± 0.25 |
| dTMP | 32.4 | 0.45 ± 0.12 |
| FWHM: Full Width at Half Maximum | | |

4. DNA Oligomer Nucleic Acid Sequence Determination

Similar tests to those of the tests in "3. Reference Nucleotide Maximum Current Value and Pulse Continuation Duration Measurement" are performed with DNA oligomers substituted for nucleic acid monomers (more specifically TGT, GTG, ATA, CAC and GAG).

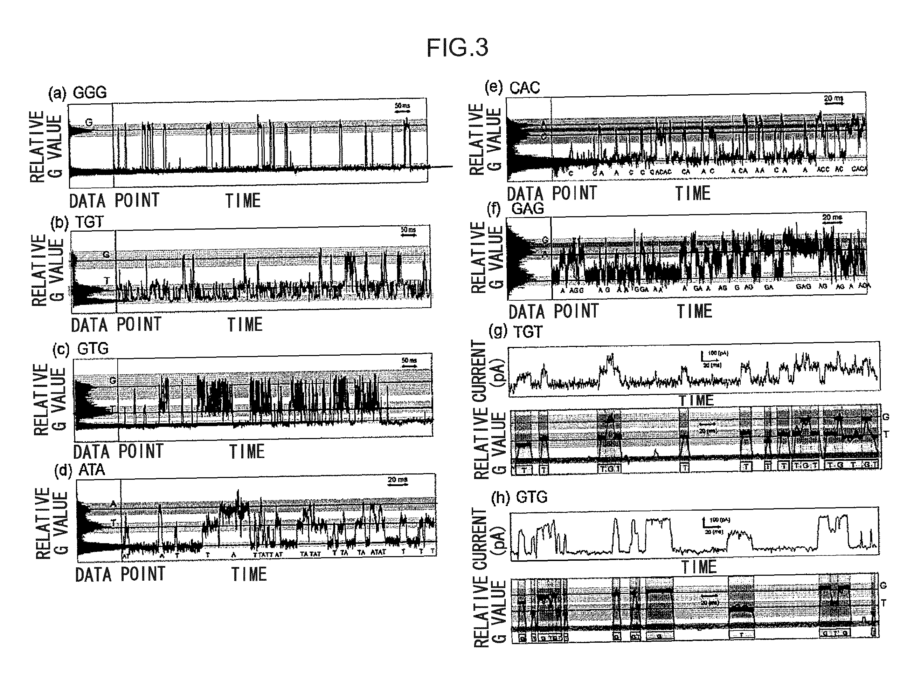

As illustrated in Fig. 3(b) to Fig. 3(f), 2 types of conductance levels are observed for each of the TGT, GTG, ATA, CAC and GAG.

In the TGT, GTG and GAG (respectively corresponding to Fig. 3(b), (c) and (f)), when the higher relative G value is taken to correspond to dGMP (=1), then the lower relative G values are respectively "0.29 ± 0.12", "0.35 ± 0.12" and "0.68 ± 0.12" (see Table 3 below).

Since these values fall within the ranges of the reference nucleotide dTMP "relative G value ± FWHM" 0.45 ± 0.12) and the reference nucleotide dAMP "relative G value ± FWHM" (0.77 ± 0.20), the obtained values of 0.29 ± 0.12, 0.35 ± 0.12 and 0.68 ± 0.12 respectively correspond to dTMP, dTMP and dAMP.

Similarly, for ATA and CAC (respectively corresponding to Fig. 3(d) and (e)), taking the higher relative G value to correspond to dAMP (= 0.77), then the lower relative G values are respectively "0.41 ± 0.07" and "0.52 ± 0.12" (see Table 3 below).

Since these values fall within the ranges of the reference nucleotide dTMP "relative G value ± FWHM" 0.45 ± 0.12) and the reference nucleotide dCMP "relative G value ± FWHM" (0.69 ± 0.25), the obtained values of 0.41 and 0.52 respectively correspond to dTMP and dCMP.

From the above results it is clear that the type of the nucleotides configuring the DNA oligomers can be obtained by determination based on the "relative G value ± FWHM" of the reference nucleotide.

| | Nucleic acid Monomer "Relative G Value ± FWHM" | GTG "Relative G Value ± FWHM" | TGT "Relative G Value ± FWHM" | ATA "Relative G Value ± FWHM" | CAC "Relative G Value ± FWHM" | GAG "Relative G Value ± FWHM" |
|---|---|---|---|---|---|---|
| dGMP | 1.00 ± 0.25 | 1.00 ± 0.29 | 1.00 ± 0.18 | - | - | 1.00 ± 0.19 |
| dAMP | 0.77 ± 0.20 | - | - | 0.77 ± 0.18 | 0.77 ± 0.14 | 0.68 ± 0.12 |
| dCMP | 0.69 ± 0.25 | - | - | - | 0.52 ± 0.12 | - |
| dTMP | 0.45 ± 0.12 | 0.35 ± 0.12 | 0.29 ± 0.12 | 0.41 ± 0.07 | - | - |

5. Pulse Continuation Duration Analysis

td (pulse continuation duration) is measured for pulses (about 1000 individual pulses) of tunnel current for dGMP and DNA oligomers (specifically GGG) and a distribution of the td observed.

Fig. 3(a) illustrates pulses of tunnel current of DNA oligomer arising over the passage of time. As illustrated in Fig. 3(a), with the DNA oligomer, similarly to dGMP, a pulse group appears with relative G values of substantially the same level.

Fig. 2(e) illustrates a distribution of td in DNA oligomers, and a distribution of td of dGMP. It is clear from Fig. 2(e) that a peak value of td of DNA oligomer is about 0.8ms, and the peak value of td of dGMP is also about 0.8ms.

From this it is possible to infer that pulses of tunnel current with td of about 0.8 to about 1ms correspond to actual nucleotides. Namely, tunnel current pulses with td shorter than these values can be inferred to be noise or the like.

For example, the tunnel current pulses corresponding to G or T are pulses with peaks with a single plateau profile, pulses with tunnel current corresponding to GT or TG are pulses with peaks with a double plateau profile, and tunnel current pulses corresponding to TGT and GTG are pulses with peaks with a triple plateau profile. Namely, in order to identify electrical signals of DNA oligomer made up from 3 nucleic acid monomers, pulse groups with a td total of 1ms or greater, and with peaks with a triple plateau profile may be identified with successive td for each of the pulses.

Fig. 3(d) to Fig. 3(f) respectively illustrate results of automatically extracting electrical signals. In these figures, clear pulses with triple plateau profile peaks are observed.

For example, in Fig. 3(g) illustrating data related to the DNA oligomer TGT, pulses detected at the first position and the third position have low plateau profile peaks representing T, and a pulse detected at the second position has a high plateau profile peak representing G.

Moreover, in Fig. 3(h) illustrating data related to the DNA oligomer GTG, pulses detected at the first position and the third position have high plateau profile peaks representing G, and a pulse detected at the second position has a low plateau profile peak representing T.

Namely, these data illustrate that the present invention can determine the base sequence of a DNA oligomer.

As illustrated in Fig. 3(g) and Fig. 3(h), in this test, not only are the sequences "GTG" and "TGT" identified, but the sequences of "G", "T", "TG", "GT", "GTGTT" and "TGTGT" are also identified. This is thought to be because as the DNA oligomer is performing Brownian motion, trapping between the nano-gap electrodes occurs probabilistically. For example, when the motion direction of the TGT oligomer due to Brownian motion between the nano-gap electrodes reverses at the third T position of the TGT oligomer, a tunnel current pulse corresponding to "TGTGT" is detected.

6. Polynucleotide Base Sequence Determination

Similar tests are performed to the tests of "5. Pulse Continuation Duration Analysis" with "5'- UGAGGUA -3'" (also referred to below as miRNA") employed in place of the DNA oligomer.

First, an I - t curve is generated in order to obtain sequence data of random fragments.

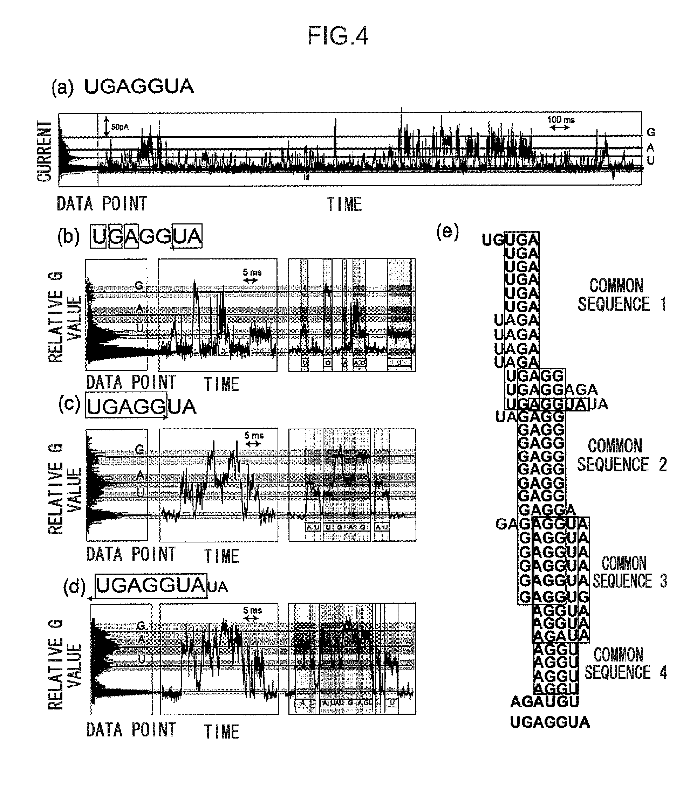

As illustrated in Fig. 4(a), in a conductance histogram produced from the I - t curve, 3 peaks appear at I = 70pS, I = 50pS, and I = 33pS.

The relative G values of these 3 peaks are respectively 1, 0.71 and 0.47, and these values respectively correspond to rGMP, rAMP and rUMP (see Table 4).

| | Reference value | Fig. 4(b) | Fig. 4(c) | Fig. 4(d) |
|---|---|---|---|---|
| rGMP | 1.00 ± 0.44 | 1.00 ± 0.16 | 1.00 ± 0.13 | 1.00 ± 0.13 |
| rAMP | 0.75 ± 0.27 | 0.65 ± 0.15 | 0.55 ± 0.16 | 0.73 ± 0.16 |

(continued)

|  | Reference value | Fig. 4(b) | Fig. 4(c) | Fig. 4(d) |
|---|---|---|---|---|
| rUMP | $0.41 \pm 0.10$ | $0.33 \pm 0.07$ | $0.29 \pm 0.11$ | $0.36 \pm 0.09$ |

The above results illustrate that there are 3 types of nucleic acid monomer contained within miRNA.

The partial base sequence of miRNA is determined similarly to in the "5. Pulse Continuation Duration Analysis" described above.

Fig. 4(b) to Fig. 4(d) illustrate typical detected signals. As illustrated in Fig. 4(b) to Fig. 4(d), "A", "G", "U", "AU", "UGAGG" and "UGAGGUA" can be determined as partial base sequences.

Similarly, analyzing 133 individual signals gives 19 individual signals corresponding to "A", 15 individual signals corresponding to "G", 44 individual signals corresponding to "U", 5 individual signals corresponding to "UA", 10 individual signals corresponding to "GA" 5 individual signals corresponding to "UG", and 35 individual signals corresponding to the sequence illustrated in Fig. 4(e).

As illustrated in Fig. 4(e), sequence data of "GAGAGGUA", "UGAGGAGA" and "UGAGGUAUA" are obtained. This sequence data is thought to arise as a result of Brownian motion.

Moreover, as illustrated in Fig. 4(e), there are occasions when "AGGUA" and "GAGGUA" in miRNA are misidentified as "AGAUA" and "GAGGUG". This may be thought to be because an overlap arises between the relative G values of rGMP and the relative G values of rAMP.

Next, as illustrated in Fig 4(e), the full base sequence of miRNA is determined by joining together repeated portions of the 35 individual partial base sequences obtained. Specifically, partial base sequences are extracted that have a high appearance frequency, and a full base sequence of miRNA is determined by joining together the partial base sequences.

Specifically, as illustrated in Fig. 4(e), 13 individual partial base sequences corresponding to "UGA" are detected out of the 35 individual partial base sequences (13/35 = 37%), 17 individual partial base sequences corresponding to "GAGG" out of the 35 individual partial base sequences (17/35 = 49%), 10 individual partial base sequences corresponding to "AGGUA" out of the 35 individual partial base sequences (10/35 = 29%), and 13 individual partial base sequences corresponding to "AGGU" out of the 35 individual partial base sequences (13/35 = 37%).

Then "UGAGGUA" is successfully determined as the full base sequence by joining together the "UGA", "GAGG", "AGGUA" and "AGGU" at the locations of repeated base sequences.

The present invention is not limited to each of the configurations explained above, and various modifications are possible within the range defined by the scope of the patent claims, and exemplary embodiments obtained by appropriate combination of the technical means described herein in each of the different respective exemplary embodiments and examples are included in the technical scope of the present invention.

Industrial Applicability

The present invention may be utilized as a device for performing polynucleotide base sequence determination by determining the tunnel current arising from one molecule of polynucleotide. The present invention may also be utilized as a device (mutation detection device) for detecting mutations (for example substitution of one base) arising in the sequences of a known polynucleotide.

The present invention is a foundation stone for next generation sequencers being pursued by the National Institutes for Health (NIH), and may be applied to next generation sequencers in which DNA amplification by PCR and chemical modification of DNA is not required. The present invention may also be applied to high sensitivity sensors for detecting a biomolecule such as an influenza virus or an allergen using one molecule thereof. Explanation of the Reference Numerals

10    VOLTAGE APPLICATION SECTION
20    ELECTRODE PAIR
30    MEASUREMENT SECTION
40    PRIMARY BASE SEQUENCE DATA GENERATION SECTION
50    SECONDARY BASE SEQUENCE DATA EXTRACTION SECTION
60    COMMON SEQUENCE SEARCH SECTION
70    SEQUENCE DATA CONNECTION SECTION
80    DATA STORAGE SECTION
100    DEVICE

**Claims**

1. A polynucleotide base sequence determination method comprising:

   a first process of passing a polynucleotide between an electrode pair;
   a second process of detecting a plurality of pulses of tunnel current arising as the polynucleotide passes between the electrode pair, and of measuring a maximum current value and pulse continuation duration for each of the plurality of pulses;
   a third process of generating primary base sequence data in which each of the plurality of pulses is associated with a specific type of nucleotide by comparing a magnitude order between the maximum current values of the plurality of pulses and a magnitude order between reference current values corresponding to electron states caused by energy level differences between individual nucleotides and metal configuring the electrode pair;
   a fourth process of extracting from among the plurality of pulses a pulse group configured by pulses with successive pulse continuation durations, and of extracting from among the primary base sequence data a plurality of secondary base sequence data corresponding to the pulse group;
   a fifth process of searching the plurality of secondary base sequence data for base sequences that are common across at least two of the secondary base sequence data; and
   a sixth process of joining together the secondary base sequence data that have the common base sequence via the common base sequence.

2. The method of claim 1, wherein:

   the third process further comprises generating the primary base sequence data in which each of the plurality of pulses are associated with a specific type of nucleotide by comparing the maximum current value against reference current values corresponding to individual nucleotides.

3. The method of claim 1 or claim 2, wherein:

   the reference current values are modal values among the maximum current values of the plurality of pulses of tunnel current arising when the nucleotides are individually passed between the electrode pair.

4. The method of claim 3, wherein:

   the electrode pair are gold electrodes; and
   the magnitude order of the reference current values when the nucleotides are DNA is dTMP < dCMP < dAMP < Methyl dAMP < dGMP < Oxo-dGMP < Methyl dCMP, and the magnitude order of the reference current values when the nucleotides are RNA is rUMP < rCMP < rAMP < rGMP.

5. The method of any one of claim 1 to claim 4, wherein:

   the sixth process extracts as tertiary base sequence data a plurality of sequence data of a base sequence found to be common in the fifth process, and joins together the tertiary base sequence data.

6. The method of any one of claim 1 to claim 5, wherein:

   the common base sequence found in the fifth process is a sequence that is common across at least 10 individual secondary base sequence data.

7. The method of any one of claim 1 to claim 6, wherein:

   in the fourth process, a plurality of secondary base sequence data are extracted that correspond to a pulse group configured from pulses with successive pulse continuation duration over a period of time of 1ms or longer.

8. The method of any one of claim 1 to claim 7, wherein:

   the electrode pair is an electrode pair in which an inter-electrode distance is maintained constant; and
   in the fourth process, the secondary base sequence data is extracted using a probabilistic statistical method.

9. A polynucleotide base sequence determination device comprising:

an electrode pair that has an inter-electrode distance through which a polynucleotide is capable of passing;
a measurement section that detects a plurality of pulses of tunnel current arising as the polynucleotide passes between the electrode pair, and that measures a maximum current value and pulse continuation duration for each of the plurality of pulses;
a primary base sequence data generating section that generates primary base sequence data in which each of the plurality of pulses is associated with a specific type of nucleotide by comparing a magnitude order between the maximum current values of the plurality of pulses and a magnitude order between reference current values corresponding to electron states caused by energy level differences between individual nucleotides and metal configuring the electrode pair;
a secondary base sequence data extraction section that extracts from among the plurality of pulses a pulse group configured by pulses with successive pulse continuation durations, and that extracts from among the primary base sequence data a plurality of secondary base sequence data corresponding to the pulse group;
a common sequence search section that searches the plurality of secondary base sequence data for base sequences that are common across at least two of the secondary base sequence data; and
a sequence data connection section that joins together the secondary base sequence data that have the common base sequence via the common base sequence.

10. The polynucleotide base sequence determination device of claim 9, wherein:

the primary base sequence data generating section further generates the primary base sequence data in which each of the plurality of pulses are associated with a specific type of nucleotide by comparing the maximum current value against reference current values corresponding to individual nucleotides.

11. The device of claim 9 or claim 10, wherein:

the reference current values are modal values among the maximum current values of the plurality of pulses of tunnel current arising when the nucleotides are individually passed between the electrode pair.

12. The device of claim 11, wherein:

the electrode pair are gold electrodes; and
the magnitude order of the reference current values when the nucleotides are DNA is dTMP < dCMP < dAMP < Methyl dAMP < dGMP < Oxo-dGMP < Methyl dCMP, and the magnitude order of the reference current values when the nucleotides are RNA is rUMP < rCMP < rAMP < rGMP.

13. The device of any one of claim 9 to claim 12, wherein:

the sequence data connection section extracts as tertiary base sequence data a plurality of sequence data of a base sequence found to be common by the common sequence search section, and joins together the tertiary base sequence data.

14. The device of any one of claim 9 to claim 13, wherein:

the common base sequence found by the common sequence search section is a sequence that is common across at least 10 individual secondary base sequence data.

15. The device of any one of claim 9 to claim 14, wherein:

the secondary base sequence data extraction section extracts a plurality of secondary base sequence data that correspond to a pulse group configured from pulses with successive pulse continuation duration over a period of time of 1ms or longer.

16. The device of any one of claim 9 to claim 15, wherein:

the electrode pair is an electrode pair in which the inter-electrode distance is maintained constant; and
the secondary base sequence data extraction section extracts the secondary base sequence data using a

probabilistic statistical method.

FIG.1

# FIG.2

(a)

(b)

(c)

(d)

(e)

FIG.3

(a) GGG

(b) TGT

(c) GTG

(d) ATA

(e) CAC

(f) GAG

(g) TGT

(h) GTG

# FIG.4

(a) UGAGGUA

(b) UGAGGUA

(c) UGAGGUA

(d) UGAGGUAUA

(e)

## FIG.5

DEVICE 100

10 VOLTAGE APPLICATION SECTION

20 ELECTRODE PAIR

30 MEASUREMENT SECTION

40 PRIMARY BASE SEQUENCE DATA GENERATION SECTION

80 DATA STORAGE SECTION

50 SECONDARY BASE SEQUENCE DATA EXTRACTION SECTION

60 COMMON SEQUENCE SEARCH SECTION

70 SEQUENCE DATA CONNECTION SECTION

RESULT

## FIG.6

```
                    ┌─────────┐
                    │  START  │
                    └────┬────┘
                         │
┌────────────────────────────────────────────────────┐
│ FILL POLYNUCLEOTIDE LIQUID BETWEEN ELECTRODES        │── S101
│          OF ELECTRODE PAIR 20                         │
└────────────────────────┬─────────────────────────────┘
                         ►◄
┌────────────────────────────────────────────────────┐
│  APPLY VOLTAGE TO ELECTRODE PAIR 20 USING            │── S102
│       VOLTAGE APPLICATION SECTION 10                  │
└────────────────────────┬─────────────────────────────┘
┌────────────────────────────────────────────────────┐
│   MEASURE MAXIMUM CURRENT VALUE AND PULSE            │── S103
│ CONTINUATION DURATION USING MEASUREMENT SECTION 30    │
└────────────────────────┬─────────────────────────────┘
┌────────────────────────────────────────────────────┐
│   GENERATE PRIMARY BASE SEQUENCE DATA USING          │── S104
│ PRIMARY BASE SEQUENCE DATA GENERATION SECTION 40      │
└────────────────────────┬─────────────────────────────┘
```

S105

IS IT POSSIBLE
TO EXTRACT PLURAL SECONDARY
BASE SEQUENCE DATA USING SECONDARY
BASE SEQUENCE DATA EXTRACTION
SECTION 50?

NO

YES

S106

IS IT POSSIBLE
TO FIND A BASE SEQUENCE COMMON
TO PLURAL SECONDARY BASE SEQUENCE
DATA USING COMMON SEQUENCE
SEARCH SECTION 60?

NO

YES

```
┌────────────────────────────────────────────────────┐
│  CONNECT TOGETHER BASE SEQUENCE DATA USING           │── S107
│    SEQUENCE DATA CONNECTION SECTION 70                │
└────────────────────────┬─────────────────────────────┘
                    ┌─────┴────┐
                    │   END    │
                    └──────────┘
```

**INTERNATIONAL SEARCH REPORT**

| International application No. |
| --- |
| PCT/JP2013/059645 |

**A. CLASSIFICATION OF SUBJECT MATTER**
*G01N27/00*(2006.01)i, *C12N15/00*(2006.01)n

According to International Patent Classification (IPC) or to both national classification and IPC

**B. FIELDS SEARCHED**

Minimum documentation searched (classification system followed by classification symbols)
G01N27/00-27/24, C12N15/00

Documentation searched other than minimum documentation to the extent that such documents are included in the fields searched
Jitsuyo Shinan Koho        1922-1996    Jitsuyo Shinan Toroku Koho    1996-2013
Kokai Jitsuyo Shinan Koho   1971-2013    Toroku Jitsuyo Shinan Koho    1994-2013

Electronic data base consulted during the international search (name of data base and, where practicable, search terms used)
JSTPlus/JMEDPlus/JST7580(JDreamII)

**C. DOCUMENTS CONSIDERED TO BE RELEVANT**

| Category* | Citation of document, with indication, where appropriate, of the relevant passages | Relevant to claim No. |
| --- | --- | --- |
| A | WO 2011/108540 A1  (Osaka University), 09 September 2011 (09.09.2011), entire text; all drawings (Family: none) | 1-16 |
| A | Michael Zwolak, Colloquium: Physical approaches to DNA sequencing and detection, REVIEWS OF MODERN PHYSICS, 2008, Vol.80, pp.141-165 | 1-16 |
| A | Masateru TANIGUCHI, "Ichibunshi Kaiseki Gijutsu ni yoru Jijisedai DNA Sequencer no Kaihatsu", Dai 69 Kai Hyomen Kagaku Kenkyukai Yoshishu, 09 March 2011 (09.03.2011), pages 23 to 26 | 1-16 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search 17 June, 2013 (17.06.13) | Date of mailing of the international search report 25 June, 2013 (25.06.13) |
| --- | --- |
| Name and mailing address of the ISA/ Japanese Patent Office | Authorized officer |
| Facsimile No. | Telephone No. |

Form PCT/ISA/210 (second sheet) (July 2009)

EP 2 833 126 A1

**INTERNATIONAL SEARCH REPORT**

International application No.

PCT/JP2013/059645

C (Continuation).   DOCUMENTS CONSIDERED TO BE RELEVANT

| Category* | Citation of document, with indication, where appropriate, of the relevant passages | Relevant to claim No. |
|---|---|---|
| A | Masateru TANIGUCHI et al., "Denryu de Ichi Enki Bunshi o Shikibetsu suru", Chemistry, 2011, vol.66, no.8, pages 42 to 46 | 1-16 |
| P,A | Masateru TANIGUCHI, "Development of Single-Molecule Bio-Nanodevices for Medical Applications", The Journal of Imaging Society of Japan, 10 February 2013 (10.02.2013), vol.52, no.1, pages 51 to 60 | 1-16 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 20111085404 A1

**Non-patent literature cited in the description**

- **J. LI ; D. STEIN ; C. MCMULLAN ; D. BRANTON ; M. J. AZIZ ; J. A. GOLOVCHENKO.** *Nature,* 2001, vol. 412, 166
- **A. J. STORM ; J. H. CHEN ; X. S. LING ; H. W. ZANDBERGEN ; C. DEKKER.** *Nature Mat.,* 2003, vol. 2, 537
- **C. DEKKER.** *Nat. Nanotechnol.,* 2007, vol. 2, 209
- **D. BRANTON ; D. W. DEAMER ; A. MARZIALI ; H. BAYLEY ; S. A. BENNER ; T. BUTLER ; M. DI VENTRA ; S. GARAJ ; A. HIBBS ; X. HUANG.** *Nat. Biotech.,* 2008, vol. 26, 1146
- **M. ZWOLAK ; M. DI VENTRA.** *Rev. Mod. Phys.,* 2008, vol. 80, 141
- *Nature Nanotechnology,* 05 April 2010, 286-290
- **J. M. VAN RUITENBEEK ; A. ALVAREZ ; I. PINEYRO ; C. GRAHMANN ; P. JOYEZ ; M. H. DEVORET ; D. ESTEVE ; C. URBINA.** *Rev. Sci. Instrum.,* 1996, vol. 67, 108
- **M. TSUTSUI ; K. SHOJI ; M. TANIGUCHI ; T. KAWAI.** *Nano Lett.,* 2008, vol. 8, 345
- **M. TSUTSUI ; M. TANIGUCHI ; T. KAWAI.** *Appl. Phys. Lett.,* 2008, vol. 93, 163115
- **TSUTSUI, M. ; SHOJI, K. ; TANIGUCHI, M. ; KAWAI, T.** Formation and self-breaking mechanism of stable atom-sized junctions. *Nano Lett.,* 2007, vol. 8, 345-349
- *Rev. Sci. Instrum.,* vol. 68 (10), 3816